(19) Europäisches Patentamt
European Patent Office
Office européen des brevets

(11) **EP 4 386 353 B1**

(12) **EUROPEAN PATENT SPECIFICATION**

(45) Date of publication and mention
of the grant of the patent:
**28.01.2026 Bulletin 2026/05**

(51) International Patent Classification (IPC):
***G01N 3/04*** (2006.01)     ***G01N 3/10*** (2006.01)

(21) Application number: **23212775.3**

(52) Cooperative Patent Classification (CPC):
**G01N 3/04; G01N 3/10;** G01N 2203/0016;
G01N 2203/0048; G01N 2203/0282;
G01N 2203/0405; G01N 2203/0411;
G01N 2203/0447

(22) Date of filing: **28.11.2023**

(54) **GRIP ASSEMBLY**

GRIFFANORDNUNG

ENSEMBLE POIGNÉE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **16.12.2022 GB 202219047**

(43) Date of publication of application:
**19.06.2024 Bulletin 2024/25**

(73) Proprietor: **Illinois Tool Works Inc.**
**Glenview IL 60025 (US)**

(72) Inventor: **MEAD, Graham**
**Glenview, 60025 (US)**

(74) Representative: **HGF**
**HGF Limited**
**4th Floor, 1 City Square**
**Leeds LS1 2AL (GB)**

(56) References cited:
**CN-U- 207 689 272      JP-A- H11 160 213**
**US-A1- 2007 107 534**

## Description

The present invention relates to a grip assembly, in particular a grip assembly for holding a sample for testing in a material testing machine.

## Introduction

Material testing apparatuses (also sometimes known as structural tests machines) are used to test the physical characteristics of a material sample. Material testing machines use a sample holder to hold the material sample and a force means to apply a testing force, via the sample holder, to test the physical characteristics of the material sample. Force means within material testing apparatuses may be actuated using a motor drive system that receives power from a power supply unit.

It is important that the sample holder is able to hold the sample with a sufficient holding force to withstand the testing force exerted on the material sample during testing.

Certain example material testing systems include a pneumatic sample holder, typically with jaws, configured to apply a holding force to hold a specimen under test.

A general drawback of grip assemblies according to the prior art is that the holding force generated by a grip assembly is restricted. That is, particularly when testing samples that withstand substantial test forces, the holding force may be inadequate to prevent sample slippage or movement during testing, generating erroneous results.

A further drawback is that the holding force exerted by sample holder on the sample is limited by the supplied pneumatic pressure, typically provided by compressed air. The pneumatic pressure supplied in an industrial or a laboratory setting has a predetermined maximum pressure, typically 6 bar, for efficiency and / or economic reasons. The supply of higher pneumatic pressure generates an increased quantity of excess heat and requires that associated equipment, such as pumps and air lines, be more robust to withstand both higher temperatures and pressures. Thus, supplied pneumatic pressures are limited to ensure acceptable operating efficiencies and to control equipment costs.

CN207689272 U, JPH11160213 A, and US 2007/107534 A1 disclose conventional pneumatic / hydraulic grips where pressure is applied inside the grip, moving the sleeve in a direction opposite to the location of the wedges, thus creating pressure on the wedges to close the grip on the sample.

It is an object of the present invention to mitigate at least some of the above problems.

Accordingly, it would be useful to provide a grip assembly that is capable of reliably holding a sample undergoing testing.

It would also be useful for a grip assembly to efficiently generate an increased holding force. That is, it would be useful for a grip assembly, to generate an increased holding force without a corresponding requirement to increase the supplied pneumatic pressure or modify the equipment associated with supplying it. In this way, it would be useful for a grip assembly to generate an increased holding force that is not limited by the supplied pneumatic pressure.

It would further be useful to provide a more compact grip assembly.

Also, it would be useful for a grip assembly to apply a holding force to a sample for testing in a manner that minimises movement of the sample.

## Summary of the Invention

The invention is set out in the appended claims.

According to an aspect of the invention, there is provided a grip assembly for holding a sample for testing in a material testing machine, including:

a core member extending along a central axis from a first end portion, configured to releasably mount the grip assembly to a crosshead, to a second end portion, wherein the core member includes a body portion having a pneumatic cylinder enclosing a pneumatic volume;

a pneumatic piston head arranged to be slidably received in the pneumatic cylinder and to be moveable along the pneumatic cylinder in a first direction to expand the pneumatic volume, and in a second, opposing, direction to contract the pneumatic volume;

a housing, including a sleeve member coaxially mounted around the body potion so that the housing is moveable relative to the core member in a first axial direction towards the first end portion and in a second, opposing axial direction;

a sample holder disposed at the second end portion, configured to selectively apply a holding force to a sample for testing; and

a driving member configured to contactingly engaging the sample holder;

wherein the grip assembly is configured so that, in response to the pneumatic piston head moving in the first direction, the housing moves in the first axial direction thereby urging the driving member against the sample holder to provide the holding force.

The grip assembly provides an effective holding force with movement of the housing in the axial direction, away from the sample holder. In this way, the grip assembly is a compact arrangement that requires reduced clearance within the testing machine. Furthermore, the holding force is applied to the sample holder while the sample holder, as part of the core member that is mounted to the crosshead, is held static with respect to the crosshead. A sample may thereby be loaded into the testing machine with a reliable spacing between the respective sample holders of the crossheads.

Aptly, the pneumatic cylinder is oriented to be coaxial with the central axis of the core member so that the first direction of movement of the pneumatic piston head is in the first axial direction, and the second direction of movement is in the second axial direction.

Aptly, the pneumatic piston head is provided on a head member configured to move relative to the core member in the first direction and the second direction.

Aptly, the grip assembly further includes a hydraulic system operably engaged with both the pneumatic cylinder and the pneumatic piston head and configured to provide a force multiplier. That is, in response to the pneumatic force provided by movement of the pneumatic piston head in the first direction, the hydraulic system generates an increased output force. The output force, also known as the output hydraulic force, is a multiple of the pneumatic force.

Aptly, the hydraulic system has a primary hydraulic cylinder formed in the housing, wherein the primary hydraulic cylinder encloses a primary hydraulic volume, and wherein the housing is configured to engage with the body portion so that the primary hydraulic volume expands when housing moves relative to the core member in the first axial direction and contracts when the housing moves relative to the core member in the second, opposing, axial direction.

Aptly, the primary hydraulic cylinder has a primary cross-sectional area, preferably defined by a base of the housing.

Aptly, the primary hydraulic cylinder is oriented to be coaxial with the central axis of the core member.

Aptly, the hydraulic system further includes at least one secondary hydraulic cylinder, enclosing a secondary hydraulic volume that is fluidly connected to the primary hydraulic volume, wherein a hydraulic piston head is arranged to be slidably received in each of the at least one secondary hydraulic cylinders and to be moveable therealong in a third direction to contract the secondary hydraulic volume, and in a fourth, opposing, direction to expand the secondary hydraulic volume.

Aptly, as the pneumatic piston head moves in the first direction, each hydraulic piston head moves in the third direction thereby contracting the secondary hydraulic volume and expanding the primary hydraulic volume.

Aptly, the hydraulic piston heads are configured to move in the first direction a distance in a range from 5mm to 60mm and, preferably, in a range from 20mm to 50mm.

Aptly, each of the at least one secondary hydraulic cylinder is formed through the body portion.

Aptly, the head member has the at least one hydraulic piston head mounted thereto, preferably releasably mounted thereto, so that the at least one hydraulic piston head is arranged to project from the head member a direction towards the first end portion of the core member.

Aptly, the at least one secondary hydraulic cylinder includes a plurality of secondary hydraulic cylinders, preferably from 2 to 8 secondary hydraulic cylinders and, more preferably, from 3 to 6 secondary hydraulic cylinders.

Aptly, each of the at least one secondary hydraulic cylinders includes a secondary central axis, and wherein each secondary central axis is arranged parallel to the central axis of the core member.

Aptly, the hydraulic system includes at least two secondary hydraulic cylinders wherein the respective secondary central axes are arranged circumferentially around the central axis of the core member.

Aptly, the at least one secondary hydraulic cylinders cumulatively provide a secondary cross-sectional area, and wherein a ratio of the primary cross-sectional area : the secondary cross-sectional area is in a range of from 1:2 to 1:20 and, preferably, in a range of from 1:5 to 1:10.

The output hydraulic force from the hydraulic system may be a multiple of the pneumatic force generated by the compressed air supplied to the pneumatic cylinder. Accordingly, an increased output hydraulic pressure is provided to the driving member. The output hydraulic force may be a multiple that is in a range from two times to twenty times, preferably in a range from five times to ten times the pneumatic force. In certain examples, the multiple may be at least two times, preferably at least five times, or more preferably at least seven times. In certain examples the multiple may be up to ten times, or up to twenty times.

Aptly, the sample holder is mounted to the second end portion of the core member and the driving member extends from the housing to contactingly engage the sample holder. The second end portion is configured so that the sample holder is mounted, preferably removably mounted, thereto.

Aptly, the housing and the body portion are configured to prevent relative rotational movement between the housing and the core member. Aptly, the body portion has a post mounted to, and projecting from, the body portion. The post is received within a recess on an inner surface of the housing that extends into the housing in a direction parallel to the central axis. In this way, as the housing moves relative to the core member, the post is rotationally constrained within the recess, preventing relative rotation between the housing and the core member. In alternative arrangements, the location of the post and the recess may be switched between the housing and the core member. Or, again, alternative features may suitably be provided in order prevent relative rotation.

Aptly, the sample holder includes a mutually-opposed pair of jaws. The at least one of the pair of jaws may be configured to slidably move in a direction transverse to the central axis.

Aptly, the sample holder is mounted to the driving member and is configured to abut the second end portion so that the second end portion prevents the sample holder moving with the driving member in first axial direction. In this way, although the sample holder is mounted to the driving member, the sample holder remains axially static as it applies a holding force to the sample for testing. The second end portion acts as a stop so that movement of the driving member in the first axial direction urges the sample holder to apply a holding force, for example by urging a mutually-opposed pair of jaws towards one another.

Aptly, the sample holder includes a pre-set spacing between the pair of jaws prior to applying the holding force to the sample. Aptly, the pre-set spacing is selectively adjustable. Aptly, the pre-set spacing is selectively adjustable between a minimum pre-set spacing and a maximum pre-set spacing.

Aptly, the driving member includes a mutually-opposed pair of engaging faces, preferably wherein each engaging face is angled relative to the central axis in a range from 10° to 45°. Each engaging face is configured to contactingly engage the sample holder and thereby urge the sample holder to provide the holding force.

Aptly, the core member includes a spacer element disposed between the second end portion and the body portion. In certain examples, where the core member includes a spacer element, the housing may include a secondary sleeve coaxially mounted around the spacer element. The secondary sleeve extends to the driving member from the sleeve mounted to the body portion. In this way, as the housing moves relative to the core member then the secondary sleeve moves relative to the spacer element, thereby urging the driving member against the sample holder.

Aptly, the axial length of a spacer element is selectively adjustable. Aptly the spacer element includes a shaft movably mounted to the body portion, and configured to move from a first axial position relative to the body portion, to a second axial position relative to the body portion. In this way, the spacer element provides an adjustable axial separation between the sample holder and the body portion of the core member. The sample holder is thus moved axially relative to the mutually-opposed angled engaging faces of the driving member. In the second axial position, the sample holder engages portions of the engaging faces at a point where the portions are set further apart than at the first axial position. The pre-set spacing between the jaws is correspondingly increased, thereby providing a selectively adjustable pre-set spacing between the pair of jaws.

Aptly, the spacer element includes a first stop element to limit the axial separation between the sample holder and the body portion of the core member to a maximal axial separation.

Aptly, the spacer element includes a second stop element to limit the axial separation between the sample holder and the body portion of the core member to a minimal axial separation.

Aptly, the housing is configured to move relative to the core member in first axial direction for a distance in a range of from 1mm to 10mm and, preferably, in a range of from 2mm to 7mm.

Aptly, the grip assembly further includes a biasing means configured to move the housing relative to the core member in a direction towards the second end portion.

Aptly, the biasing means includes either or both of: a pneumatic release chamber and at least one spring. In examples where the biasing means includes a pneumatic release chamber, the chamber may be fluidly connected to a second mains supply of compressed air. The grip assembly includes a second conduit configured to fluidly connect a first release port, connectable to the second mains supply of compressed air, to a second release port that opens into the pneumatic release chamber.

Aptly, the grip assembly includes a conduit configured to fluidly connect a first port, connectable to a mains supply of compressed air, to a second port that opens into the pneumatic cylinder.

Aptly, the compressed air from one or both of the mains supply and the second mains supply may be conveyed into the grip assembly through the core member. The first port and / or the first release port, is provided on the core member at, or proximal to, the first end portion. The conduit and / or the second conduit extends along the central axis of the core member to the second port and / or second release port. Preferably, a connector is mounted within the first port and / or the first release port to enable the port to connect to an air-line of the respective mains supply of compressed air.

Aptly, the pneumatic piston head is provided on a head member, moveable relative to the core member, and wherein the head member includes a tube arranged to extend through the head member from the second port towards the first end portion so as to telescopingly engage with the conduit. The tube is slidably received within a portion of the conduit. In this way, the second port is provided on the head member and is fluidly connected to the conduit in a compact and convenient arrangement. The conduit remains fluidly connected to the pneumatic cylinder as head member moves relative to the conduit.

Aptly, the first port is arranged on the core member and aligned with an opening extending through the sleeve mounted around the core member, and wherein the opening is configured so that the first port remains accessible through the opening as the housing moves relative to the core member.

Certain examples urge the sample holder to provide a holding force that exceeds, and preferably is a multiple of, a holding force achievable by the pneumatic pressure supplied to the grip assembly by the compressed air supply.

Certain examples achieve a holding force by urging the sample holder to hold the sample with a force that is significantly greater than would be achieved solely by the pneumatic pressure from the compressed air supplied to the grip assembly.

Certain examples provide a more reliable holding force. In particular the holding force enables testing of a sample using increased mechanical forces without risk of the sample slipping from the sample holder.

Certain examples provide a more compact grip assembly. In particular, a force multiplier is provided within the grip assembly in a compact configuration.

Certain examples provide a holding force more efficiently because a reliable holding force is achieved using compressed air that is supplied at a lower pressure.

## Brief Description of the Drawings

Embodiments of the invention are now described, by way of example only, hereinafter with reference to the accompanying drawings, in which:

**Figure 1** shows a perspective view of a known material testing apparatus;

**Figure 2** shows a perspective view of an example grip assembly;

**Figure 3** shows a cross-sectional view of the example grip assembly of the example of Figure 2;

**Figure 4** shows (a) upper and (b) lower perspective views of a sub-assembly of the core member of the example of Figure 2;

**Figure 5** shows a lower perspective view of a sub-assembly of the example of Figure 2;

**Figure 6** shows a lower perspective view of a further sub-assembly of the example of Figure 2

**Figure 7** shows a cross-sectional view of another example grip assembly;

**Figure 8** shows a perspective view of a further example grip assembly alongside a magnified perspective view of a portion of the assembly;

**Figure 9** shows a cross-sectional view of another example grip assembly;

**Figure 10** shows a perspective view of a further example grip assembly;

**Figure 11** shows a cross-sectional view of the example grip assembly of Figure 10;

**Figure 12** shows a cross-sectional view of another grip assembly;

**Figure 13** shows a cross-sectional view of a yet further example grip assembly; and

**Figure 14** shows cross-sectional views of yet another example grip assembly in which the pre-set spacing of the pair of jaws is (a) at a maximal spacing, and (b) at a minimal spacing.

In the drawings, like reference numerals refer to like parts.

## Detailed Description

Certain terminology is used in the following description for convenience only and is not limiting. The words 'lower' and 'upper' designate directions in the drawings to which reference is made and are with respect to the described component when assembled and mounted. The words 'inner', 'inwardly' and 'outer', 'outwardly' refer to directions toward and away from, respectively, a designated centreline or a geometric centre of an element being described (e.g. central axis), the particular meaning being readily apparent from the context of the description.

Further, as used herein, the terms 'connected', 'coupled', and 'mounted' are intended to include direct connections between two members without any other members interposed therebetween, as well as, indirect connections between members in which one or more other members are interposed therebetween. The terminology includes the words specifically mentioned above, derivatives thereof, and words of similar import.

Further, unless otherwise specified, the use of ordinal adjectives, such as, 'first', 'second', 'third', 'primary', 'secondary' etc. merely indicate that different instances of like objects are being referred to and are not intended to imply that the objects so described must be in a given sequence, either temporally, spatially, in ranking or in any other manner.

As used herein, a "crosshead" refers to a component of a material testing system that is used to apply a directional (axial) and/or a rotational force to a specimen. A material testing system may have one or more crossheads, and the crosshead(s) may be located in any appropriate position and/or orientation in the material testing system.

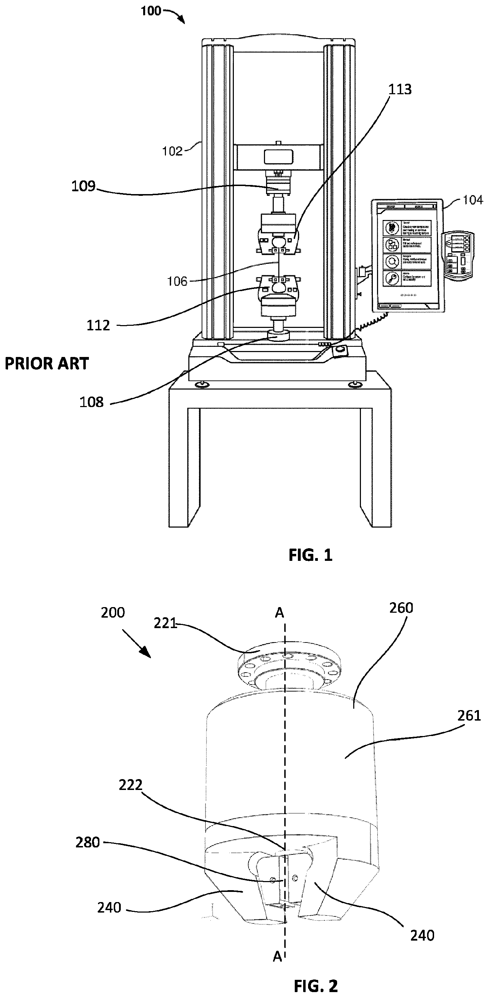

Referring now to Figure 1, there is shown an example material testing system 100 to perform mechanical property testing. The example material testing system 100 may be, for example, a universal testing system capable of static mechanical testing. The material testing system 100 may perform, for example, compression strength testing, tension strength testing, shear strength testing, bend strength testing, deflection strength testing, tearing strength testing, peel strength testing (e.g., strength of an adhesive bond), torsional strength testing, and/or any other compressive and/or tensile testing. Additionally or alternatively, the material testing system 100 may perform dynamic testing.

The example material testing system 100 includes a test fixture 102 and a computing device 104 communicatively coupled to the test fixture 102. The test fixture 102 applies loads to a sample material under test 106 and measures the mechanical properties of the test, such as displacement of the sample under test 106 and/or force applied to the material under test 106.

The example computing device 104 may be used to configure the test fixture 102, control the test fixture 102, and/or receive measurement data (e.g., transducer measurements such as force and displacement) and/or test results (e.g., peak force, break displacement, etc.) from the test fixture 102 for processing, display, reporting, and/or any other desired purposes.

In the example shown, the test fixture 102 includes a first crosshead 108 and a second crosshead 109. Each crosshead has a grip assembly 112, 113 mounted thereto. Each grip assembly is used to hold a portion of the sample under test 106 as its mechanical properties are tested. Each grip assembly 112, 113 includes sample holder, typically gripping jaws, to apply a holding force to hold the material sample. The holding force is typically provided by a pneumatic actuator.

The first crosshead 108 is a static crosshead such that the respective grip assembly 113 mounted to the first crosshead 108 remains static as loads are applied to the sample under test 106. The second crosshead 109 is a moveable crosshead and is moved within the test fixture 102 to apply a load to the sample under test 106. Movement of the second crosshead 108 is controlled by the computing device 104. The example grip assemblies described herein may be mounted to and used with both a static crosshead and a moveable crosshead.

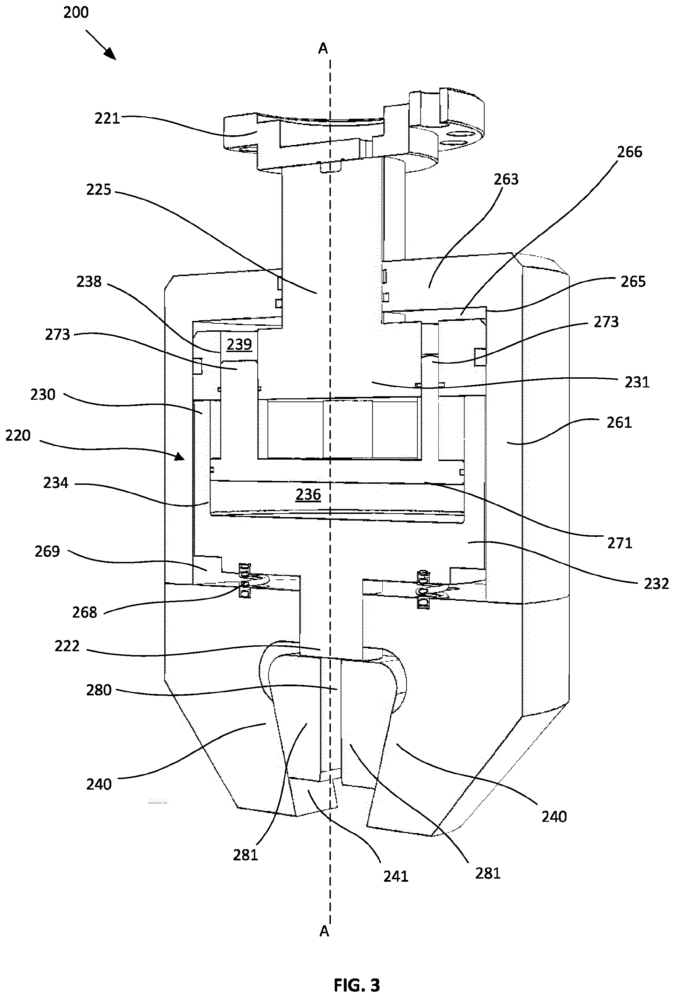

Referring now to Figures 2 and 3, there is shown an example grip assembly 200 for holding a sample for testing in a material testing machine, such as the material testing machine 100 described with reference to Figure 1.

The grip assembly 200 includes a core member 220 extending along a central axis A from a first end portion 221 to a second end portion 222, wherein the core member 220 includes a body portion 230 having a pneumatic cylinder 234 enclosing a pneumatic volume 236. The first end portion 221 is configured to releasable mount the grip assembly 200 to a crosshead (not shown), for example the static first crosshead 108 or the moving second crosshead 109 described with reference to Figure 1. The grip assembly 200 also includes a pneumatic piston head 271 arranged to be slidably received in the pneumatic cylinder 234 and to be moveable along the pneumatic cylinder 234 in a first direction to expand the pneumatic volume 236, and in a second, opposing, direction to contract the pneumatic volume 236. A housing 260 is provided, including a sleeve member 261 coaxially mounted around the body potion 230 so that the housing 260 is moveable relative to the core member 220 in a first axial direction towards the first end portion 221 and in a second, opposing axial direction. The grip assembly 200 further includes a sample holder 280 disposed at the second end portion 222, configured to selectively apply a holding force to a sample for testing, and a driving member 240 configured to contactingly engage the sample holder 280. The grip assembly 200 is configured such that, in response to the pneumatic piston head 271 moving in the first direction, the housing 260 moves in the first axial direction thereby urging the driving member 240 against the sample holder 280 to provide the holding force.

The first end portion 221 is mounted to a crosshead in any suitable known manner, for example by a locking pin inserted through co-aligned openings in the first end portion 221 and the cross head.

In this example, the pneumatic cylinder 234 of the grip assembly 200 is oriented to be coaxial with the central axis A of the core member 220. In this way, the first direction of movement of the pneumatic piston head 271 is in the first axial direction, and the second direction of movement is in the second axial direction.

The body portion 230 of the core member 220 is disposed between the first end portion 221 and the second end portion 222. In particular, the body portion 230 is disposed axially between the first end portion 221 and second end portion 222. The second end portion 222 is in a fixed position relative to the first end portion 221 because each is mounted to the body portion 230. In particular, the second end portion 222 is in a fixed axial position relative to the first end portion 221. In this way, with the first end portion 221 mounted to a crosshead, the housing 260 is moveable relative to the core member 220 and also moveable relative to each of the crosshead and the second end portion 222. Advantageously, as the housing 260 moves in the first direction to urge the driving member 240 against the sample holder 280, both the second end portion 222 and the sample holder 280 disposed at the second end portion 222 remain in a fixed position relative to the crosshead. Thus, as a sample is loaded into the sample holder 280 for testing then a holding force may be applied without axial movement of the sample holder 280.

The body portion 230 is substantially surrounded by the sleeve 261 of the housing 260. The body portion 230 includes a first body element 231 and a second body element 232. The first body element 231 and second body element 232 are configured to be selectively mounted to each other to form the body portion 230. In the example, the first body element 231 and second body element 232 are fixedly mounted together using compatible fastening bolts and threads arranged provided at suitable locations on each body element.

The core member 220 includes a first shaft 225, extending from the first body element 231 to the first end portion 221. The core member 220 also includes a second shaft 226, extending from the second body element 232 to the second end portion 222. Each of the first shaft 225 and the second shaft 226 is coaxial with the central axis A of the core member 220.

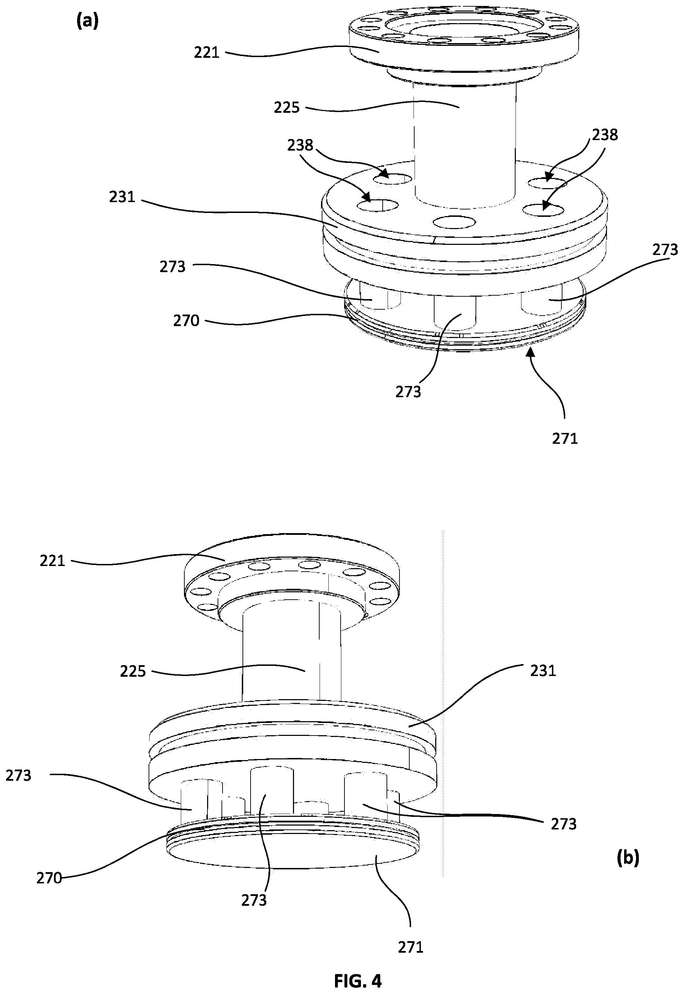

The pneumatic piston head 271 is provided on a head member 270. Referring additionally to Figures 4(a) and (b), the pneumatic piston head 271 is shown provided on the head member 270 that is operably engaged with the first body element 231 of the core member 220. The head member 270 includes a plurality, in this example six, hydraulic piston heads 273. Each hydraulic piston head 273 extends from the head member 270 in a direction of the first end portion 221 so as to be slidably received within a respective secondary hydraulic cylinder 238, as is described in further detail below. Each hydraulic piston head 273 has an axial length at least equal to the maximum distance of travel of the head member 270, as is described herein.

The head member 270 is configured to move relative to the core member in the first and second direction. The head member 270 is mounted within the body portion 230 of the core member 220 to move relative to both the core member 220 and the housing 260. Stated differently, the core member 220 forms a static support, that is static relative to the crosshead to which it is mounted, to which both the housing 260 and the head member 270 are moveably mounted so as to move independent of one another.

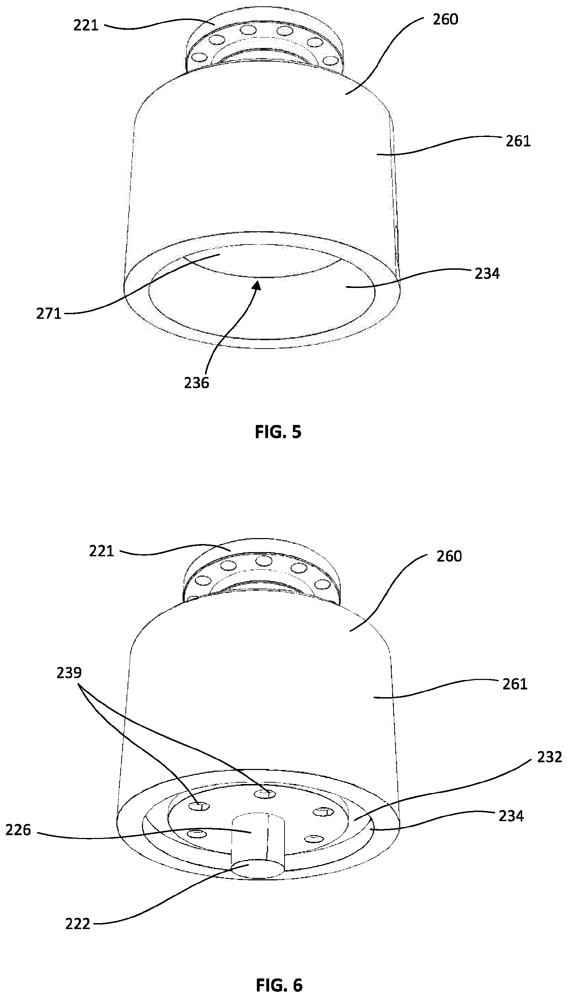

Referring additionally to Figure 5, there is shown another sub-assembly including the sub-assembly shown in Figures 4(a) and (b) operably engaged with the housing 260. In Figure 5, the driving member 240 of the housing 260 is not shown. The housing 260 includes a sleeve 261 and a base 263 disposed at a first axial end of the sleeve 261.

The base 263 extends radially inward from the sleeve 261, extending towards the first shaft 225 of the core member 220. A radially inner surface of the sleeve 261 abuts the first shaft 225. The base 263 moves relative to the first shaft 225 as the housing 260 moves relative to the core member 220.

The pneumatic piston head 271 has a cross-sectional area which, in the example, corresponds to a pneumatic cross-sectional area of the pneumatic cylinder 234 measured normal to the axis of the pneumatic cylinder 234. The pneumatic piston head 271 is a snug fit within the pneumatic cylinder so as to form a fluid tight seal with cylinder walls.

Referring in addition to Figure 6, there is shown a further sub-assembly, including the sub-assembly of Figure 5 operably engaged with the second body element 232 of the core member 220. The second body element 232 is shown arranged on the pneumatic piston head 271 to form the static, closed end of the pneumatic cylinder 234. As shown, the sleeve 261 extends around the second body element 232.

The second shaft 226 of the core member 220 extends from the second body element 232 to the second end portion 222. It will be noted that grip assembly 200 further includes the driving member 240 (not shown in Figures 4 to 6) which is mounted around the second shaft 226 to the core member 220 to be moveable, particularly axially moveable, relative thereto. In the example shown, the driving member 240 is mounted to the housing 260 at a second axial end of the sleeve 261. The second axial end of the sleeve 261 includes a closure to the housing 260. Thus, the driving member 240 is moveably mounted to the second shaft 226 in a corresponding manner to how the base 263 is moveably mounted to the first shaft 225. Movement of the housing 260 relative to the core member 220 in the first axial direction moves the driving member 240 in the first axial direction. Movement of the housing 260 in the second, opposing axial direction also moves the driving member 240 in the second axial direction.

With the housing 260 mounted to the body member 230, a pneumatic release chamber 269 is formed between the second body element 232 and the closure. In the example, the pneumatic release chamber 269 is selectively supplied with compressed air to provide a biasing means to configured to move the housing 260 relative to the core member 220 in a direction towards the second end portion 222. In this way, the biasing means act to release the holding force applied to a sample in the sample holder 280, as described in more detail herein.

The grip assembly 200 further includes a hydraulic system. The hydraulic system is operably engaged with both the pneumatic cylinder 234 and the pneumatic piston head 271 to provide a force multiplier. That is, in response to the pneumatic force provided by movement of the pneumatic piston head 271 in the first direction, the hydraulic system generates an increased output force. The output force, also known as the output hydraulic force, is a multiple of the pneumatic force. The hydraulic system is configured so that its output force urges the driving member 240 against the sample holder 280 to provide the holding force. In this way, the grip assembly 200 provides a holding force to the sample holder 280 urged by an output force which exceeds, and preferably is a multiple of, the pneumatic force generated within the grip assembly 200.

The hydraulic system includes a primary hydraulic cylinder 265 formed in the housing 260. The primary hydraulic cylinder 265 encloses a primary hydraulic volume 266. The housing 260 is configured to engage with the body portion 230 of the core member 220 so that the primary hydraulic volume 266 expands when housing 260 moves relative to the core member 220 in the first axial direction and contracts when the housing 260 moves relative to the core member 220 in the second, opposing, axial direction. In the example shown, the primary hydraulic cylinder 265 is oriented to be coaxial with the central axis of the core member 220. In addition, the primary hydraulic cylinder 265 is configured to move relative to the core member 220 in first axial direction for a distance of 3.7mm. Stated differently, the range of axial travel of the housing 260 relative to the core member 220 is 3.7mm.

The primary hydraulic cylinder 265 has a primary cross-sectional area. That is, the primary hydraulic cylinder 265 has a primary cross-sectional area measured normal to the axis of movement of the housing 260.

In the example, the primary cross-sectional area is the cross-sectional area of the inner surface of the base 263. The primary cross-sectional area is 9313mm².

The hydraulic system also includes a plurality of secondary hydraulic cylinders 238, in this example six secondary hydraulic cylinders 238. Each secondary hydraulic cylinder 238 encloses a respective secondary hydraulic volume 239 that is fluidly connected to the primary hydraulic volume 266. That is each secondary hydraulic volume 239 is fluidly connected to the primary hydraulic volume 266 via an open end of the secondary hydraulic cylinder 238.

A hydraulic piston head 275 is arranged to be slidably received in each of the secondary hydraulic cylinders 238 and is moveable therealong in a third direction to contract the secondary hydraulic volume 239, and in a fourth, opposing, direction to expand the secondary hydraulic volume 239. In the example, six hydraulic piston heads 273 are provided. Furthermore, the head member 270, and thereby each hydraulic piston head 273, is configured to move in the third direction, corresponding to the first axial direction, for a distance of 27mm. Stated differently, the range of axial travel of the head member 270 relative to the core member 220 is 27mm.

The head member 270 to which the pneumatic piston head 271 is mounted also has the hydraulic piston heads 275 mounted thereto. Each hydraulic piston head 275 is arranged to project from the head member 270 a direction towards the first end portion of the core member 220.

The hydraulic piston head 273 may be formed with the head member 270, such that the head member 270, hydraulic piston head 273 and pneumatic piston head 271 are formed as a single, unitary component. Alternatively, the pneumatic piston head and / or one or more of the hydraulic piston heads may be releasably mounted to the head member. A pneumatic piston head may be releasably mounted in any suitable manner, for example by secured to the head member using a locking nut to engage a threaded portion of the head member.

Each of the secondary hydraulic cylinders 238 includes a secondary central axis. The secondary hydraulic cylinders 238 are arranged so that each secondary central axis is in parallel to the central axis of the core member 220. Thus, the movement of each secondary hydraulic cylinder 238 in the third direction corresponds to movement in the first axial direction, and the movement of each secondary hydraulic cylinder 238 in the fourth direction corresponds to movement in the second axial direction.

The respective secondary central axes are arranged circumferentially around the central axis A of the core member 220. Correspondingly, the hydraulic piston heads 273 are arranged circumferentially around the head member 270. In the example, the secondary central axes, are spaced evenly around the central axis A and, correspondingly, the hydraulic piston heads 273 are spaced evenly around the head member 270. However, any suitable arrangement of the secondary central axes, and hydraulic piston head, may be provided. Circumferential and radial spacings may be varied between to accommodate arrangements of the secondary hydraulic cylinders and secondary hydraulic piston heads.

Each of the secondary hydraulic cylinders 238 is formed through the body portion 230 230. In particular, in the example each secondary hydraulic cylinder 238 is formed through the first body element 231. That is, the first body portion 231 includes a plurality, in this example six, secondary hydraulic cylinder 238 extending therethrough and arranged to receive a respective hydraulic piston head 273.

Each secondary hydraulic cylinder 238 has a cross-sectional area measured normal to the central axis of the secondary hydraulic cylinder 238. The secondary hydraulic cylinders 238 thereby cumulatively provide a secondary cross-sectional area corresponding to the total cross-sectional area of all the secondary hydraulic cylinders 238 of the grip assembly 200. In the example, each of the six secondary hydraulic cylinders 238 has a cross-sectional area of 201mm² providing a secondary cross-sectional area of 1206mm². Thus a ratio of the primary cross-sectional area : the secondary cross-sectional area is

$$\frac{9313}{1206} = 7.72$$

The ratio of the primary cross-sectional area : the secondary cross-sectional area is a multiplying factor to the pneumatic force applied to the pneumatic piston head 271 in order to generate the output force of the hydraulic system. In the example grip assembly 200 the multiplying factor is 7.72. The pneumatic force applied to the pneumatic piston head 271 in the pneumatic cylinder 234 is multiplied by a factor of 7.72 to generate an output force for urging the driving member 240 against the sample holder 280 to apply the holding force. The pneumatic piston head has an area of 7854mm². Compressed air at 6 bar pressure supplied to the pneumatic cylinder 234 of the grip assembly 200 generates a pneumatic force of 4.7kN which is multiplied to an output force of 36.3kN for urging the driving member 240.

The grip assembly 200 includes the sample holder 280 mounted to the second end portion 222 of the core member 220. The sample holder 280 comprises a mutually-opposed pair of jaws. The pair of jaws 281 is mounted to the second end portion 222 and configured to slidably move relative to the second end portion 222 in a direction transverse to the central axis. The jaws 281 slide towards one another, that is slide radially inwards, to apply a holding force to a sample placed between the jaws 281. The jaws 281 slide away from one another, that is slide radially outwards, to release the holding force from the sample.

The driving member 240 extends from the housing 260 to contactingly engage the sample holder 280. In the example, the driving member 240 includes a mutually-opposed pair of engaging faces 241 arranged radially outward of the pair of jaws 281. As will be apparent, the driving member 240 and each of its pair of engaging faces 241 will move as the housing 260 moves relative to the core member 220. Thus, the driving member 240 and each of the pair of engaging faces 241 move in a first axial direction, towards the first end portion 221, and in a second, opposing axial direction. In particular, with the core member 220 remaining static, then when each of the pair of engaging face 241 move in first axial direction they are moved towards both the second end portion 222 and the sample holder 280 mounted thereto. And, when the pair of engaging faces 241 move in the second axial direction, they are moved away from both the second end portion 222 and the sample holder 280.

In this way, the grip assembly is configured to provide a holding force to a sample while the sample holder 280 remains static. Furthermore, by moving the housing 260 towards the first end portion 221, a conveniently compact grip assembly is provided.

Each engaging face 241 is angled at 15° relative to the central axis A. That is each engaging face 241 faces towards the housing 260 at an angle of 15° relative to the central axis A. Each engaging face 241 is configured to contactingly engage the sample holder 280 and thereby urge the sample holder 280 to provide the holding force. In particular, each of the engaging faces 241 abuts a chamfered outer face of a jaw 281. In this way, as the driving member 240 moves in the first axial direction each engaging face 241 acts on a respective chamfered outer face to urge the jaws of the sample holder 280 radially inwards, thereby providing the holding force.

The force with which the engaging faces 241 of the driving member 240 urge the respective chamfered outer faces is dependent upon the force applied to move the housing 260 in the first axial direction. Thus, by using the hydraulic system the pneumatic force is multiplied, providing a holding force that is considerably greater than would be provided using only a pneumatic force generated from the supplied compressed air.

Due to the respective angles of the engaging face 241 and the chamfered outer faces, the range of travel of the pair of jaws due to the urging of the driving member 240 is 2mm. That is, the range of radial travel of each jaw 281 of the pair of jaws is 1mm.

Optionally, the sample holder 280 may include an adjustable pre-set spacing between the pair of jaws prior to applying the holding force to the sample. In this way, the pair of jaws may be adjusted according to the thickness of the sample they are to hold and the range of travel of the pair of jaws. The pre-set spacing may be selectively adjustable by the user so as to move the pair of jaws towards or away from one another before a holding force is applied.

As will be appreciated, the sample holder may be any suitable arrangement suitably for use with the driving means of the grip assembly. In an alternative example, in contrast to the example described with reference to Figures 2 to 6, the pair of jaws of the sample holder may be mounted to driving member. In particular, the pair of jaws may be mounted to a respective engaging so that each jaw is slidable therealong at an angle to the central axis A. The jaws each abut the second end portion so that, in use, the second end portion prevents the jaws moving with the driving member in the first axial direction. The jaws thereby move towards one another by sliding towards a distal end of each engaging face, to apply a holding force to a sample placed between the jaws. After a sample has been tested, the jaws move away from one another, that is by sliding away from the distal end of each engaging face, to release the holding force from the sample.

The grip assembly 200 further includes a conduit (not shown) configured to fluidly connect a first port, connectable to a mains supply of compressed air, to a second port that opens into the pneumatic cylinder 234. Typically, the mains supply of compressed air is selectively provided to the grip assembly by a controller operated by the user or by a computing device associated with the testing machine. The conduit and ports may be any suitable conduit arrangement such as those described with reference to any of the examples disclosed herein.

The grip assembly includes a plurality of sealing means. The sealing means provide fluid seals between the component as they move relative to one another. In this way, respective pneumatic and hydraulic volumes are sealed against leaks. The sealing means may be any suitable dimension and material as is known in the art. Typically, sealing means for pneumatic components include an O-ring. An O-ring may be a rubber O-ring. An O-ring may have a round cross-sectional profile, typically where profile has a diameter 5.3mm. Typically, sealing means for hydraulic components includes a U-cup profile. A hydraulic seal may be formed of rubber or other suitable polymer. A U-cup profile may have a cross-sectional width of 5 mm.

The grip assembly 200 further includes biasing means, such as used to release the jaws, or to move the housing 260 in the second axial direction after testing of the sample is complete. Each biasing means may include a plurality of springs 268. In the present example, a series of recesses 239 are provided in the chamber 269 above the second body element 232. Each recess 239 locatingly receives a respective spring 268 of the biasing means. When a pneumatic pressure is released from the pneumatic cylinder 234 the housing 260 ceases to receive an output force to urge the driving member 240 against the sample holder 280. Biasing means thus overcome any inertia or sticking between components and ensure that the housing 260 moves in the second axial direction, urging hydraulic fluid in the primary hydraulic cylinder towards the secondary hydraulic cylinders 238.

Additionally, or alternatively, the chamber may be a pneumatic release chamber that is selectively supplied with a pneumatic release pressure. The chamber may be configured so that a pneumatic release pressure is provided to the chamber when the pneumatic pressure is released from the pneumatic cylinder. In this way, the pneumatic release pressure acts to overcome any inertia or sticking between components and ensure that the housing moves in the second axial direction. Optionally, the biasing means may include any suitable combination of pneumatic release pressure and springs.

In use, the grip assembly 200 is mounted to a crosshead of a material testing machine and connected to a supply of compressed air. Initially compressed air is not conveyed into the grip assembly 200. The pneumatic piston head 271 is disposed in a first position so that pneumatic volume 236 is contracted to a minimum pneumatic volume 236. Consequently, the housing 260 is disposed relative to the core member 220 at maximum distance from the first end portion 221. The driving member 240 contactingly engages the sample holder 280 but is not urged against the sample holder 280. The spacing between the pair of jaws 281 of the sample holder 280 are at a maximum distance.

As will be apparent, with the pneumatic piston head 271 in the first position, the secondary hydraulic volume 239 of each secondary hydraulic cylinder 238 is expanded to a maximum. Hydraulic fluid within the hydraulic system fills each secondary hydraulic volume 239. As the hydraulic system contains a fixed volume, the primary hydraulic volume 266 is contracted to a minimum. The biasing means provide substantially the only force acting on the housing 260 to ensure the housing 260 is urged into the first position.

To load a sample for testing in a material testing machine, the sample is placed in the spacing between the pair of jaws 281. The controller is operated to provide compressed air to the grip assembly 200.

The compressed air is conveyed via the first port, the conduit and the second port to the pneumatic cylinder 234 thereby providing an increased pneumatic pressure in the pneumatic cylinder 234. The pneumatic pressure acts on the pneumatic piston head 271 to move the pneumatic piston head 271 along the pneumatic cylinder 234 in the first axial direction. The pneumatic piston head 271 provides a pneumatic force. As will be apparent the pneumatic piston head 271 will only move once the pneumatic pressure overcomes any residual force provided by biasing means in the grip assembly 200. However, typically, the residual force is relatively small compared the force provided by the pneumatic pressure, so that once a pneumatic pressure is provided from the supplied compressed air then the residual force is overcome without substantially impeding the pneumatic piston head 271.

The pneumatic piston head 271 moves in first axial direction, providing a pneumatic force, and acting on the hydraulic system to move each hydraulic piston head 273 in the first axial direction within their respective secondary hydraulic cylinder 238. Each hydraulic piston head 273 provides an increased hydraulic pressure within its respective secondary hydraulic cylinder 238 urging hydraulic fluid into the primary hydraulic cylinder 265. The primary hydraulic cylinder 265 thereby expands, causing the housing 260 to move in the first axial direction.

The housing 260 moves in the first axial direction, urging the driving member 240 against the sample holder 280, reducing the spacing between the pair of jaws 281 until each jaw contacts the sample. With the sample in contact with the jaws, the pneumatic pressure within the pneumatic cylinder 234 continues to urge the housing 260 in the first axial direction, urging the driving member 240 against the sample holder 280 to provide a holding force for holding the sample for testing.

With holding force acting on the sample, the pneumatic piston head 271 is disposed in a second position. In the second position, the pneumatic volume 236 is expanded to a maximum pneumatic volume for the sample held in the sample holder 280. Consequently, for the particular sample, the housing 260 is disposed relative to the core member 220 at minimum distance from the first end portion 221.

With the pneumatic piston head 271 in the second position, the secondary hydraulic volume 239 of each secondary hydraulic cylinder 238 is contracted to a minimum secondary hydraulic volume for the particular sample, and the primary hydraulic volume 266 is corresponding expanded to a maximum primary hydraulic volume.

When testing of the sample is complete, the controller is operated to cease conveying compressed air to the grip assembly and to release any pneumatic pressure. Thereafter the biasing means overcomes any inertia or sticking between components and ensures that the housing 260 moves in the second axial direction. The biasing means moves the housing 260, the hydraulic piston head 273 and the pneumatic piston head 271 back to their respective first positions.

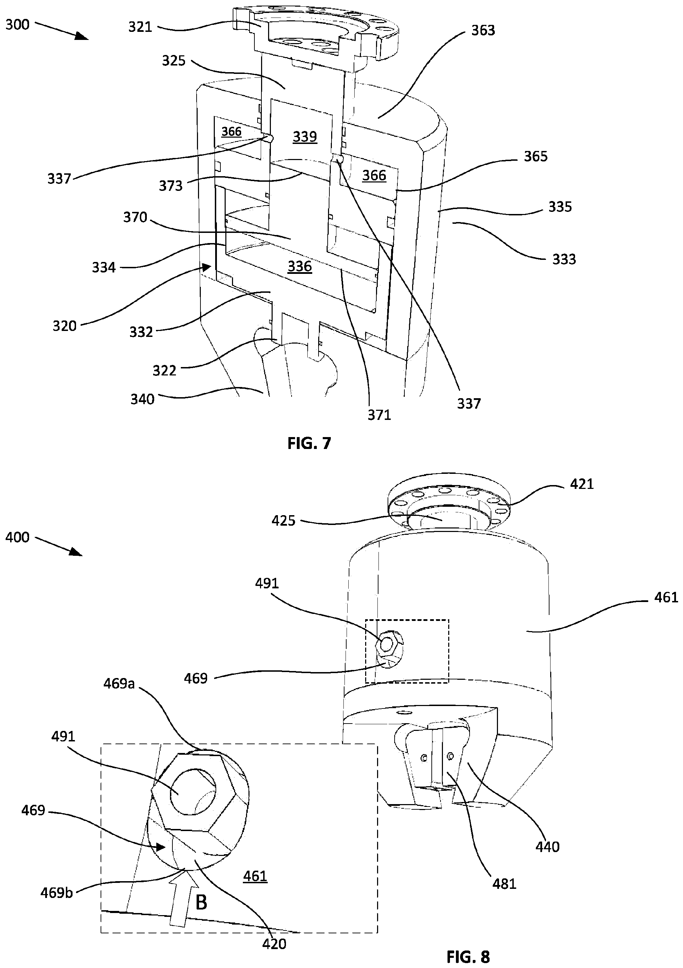

Referring now to Figure 7, there is shown another example grip assembly 300 for holding a sample for testing in a material testing machine. Where the features are the same as a previous example, the reference numerals are the same, other than the initial digit is "3". The assembly includes a core member 320, including a pneumatic cylinder 334 and pneumatic piston head 371, as well as a housing 360 substantially the same as the example described with reference to Figures 2 to 6. The hydraulic system of the grip assembly 300 provides a corresponding multiplication of the pneumatic force to generate an output force but, instead of a plurality of hydraulic piston heads and secondary hydraulic cylinders, the system has one hydraulic piston head 373 and one secondary hydraulic cylinder 338.

The pneumatic piston head 371 is mounted to a head member 370 mounted within the body portion 330 of the core member 320 to move relative to both the core member 320 and the housing 360. The head member 370 includes a hydraulic piston head 373 extending from the head member 370 in a direction of the first end portion 321 so as to be slidably received within a secondary hydraulic cylinder 338. The secondary hydraulic cylinder 338 includes a secondary central axis aligned with the central axis A of the core member 320. Thus, the hydraulic piston head 373 is arranged correspondingly to extend along the central axis A when received within the secondary hydraulic cylinder 338. The secondary hydraulic cylinder 338 has a secondary cross-sectional area measured normal to the central axis of the secondary hydraulic cylinder 338.

The secondary hydraulic cylinder 338 extends axially within the first body element 331 towards the first shaft 325. In this way, the base 363 of the housing 360 includes a radially inner surface that abuts the first shaft 325 around the secondary hydraulic cylinder 338.

A plurality of openings 337 is formed through the first shaft 325 to fluidly connect the secondary hydraulic cylinder 338 to the primary hydraulic cylinder 365. In the example, the plurality of openings 337 is a pair of radially oriented openings 337 extending through a portion of the first shaft 325 that contains the secondary hydraulic cylinder 338. The openings 337 are arranged beyond the limit of axial travel of the hydraulic piston head 373 along the secondary hydraulic cylinder 338. In this way, the openings 337 remain unblocked as the hydraulic piston head 373 moves relative to the core member 320.

In the example, the secondary hydraulic cylinders 338 has a cross-sectional area of 1075mm². The secondary cross-sectional area is also 1075mm². The primary cross-sectional area is the same as the example described with reference to Figures 2 to 6, that is 9313mm². Thus a ratio of the primary cross-sectional area : the secondary cross-sectional area is

$$\frac{9313}{1075} = 8.66$$

The ratio of the primary cross-sectional area : the secondary cross-sectional area is a multiplying factor to the pneumatic force applied to the pneumatic piston head 371 in order to generate the output force of the hydraulic system. In the example grip assembly 300 the multiplying factor is 8.66. The pneumatic force applied to the pneumatic piston head 371 in the pneumatic cylinder 334 is multiplied by a factor of 8.66 to generate an output force for urging the driving member 340 against the sample holder 380 to apply the holding force. Compressed air at 6 bar pressure supplied to the pneumatic cylinder 234 of the grip assembly 200 generates a pneumatic force of 4.7kN which is multiplied to an output force of 40.7kN for urging the driving member 340.

In use, the grip assembly operates in substantially the same way as the example described with reference to Figures 2 to 6 except that movement of the hydraulic piston head 373 in the first axial direction urges hydraulic fluid from the secondary hydraulic cylinder 338 through the plurality of openings 337 to the primary hydraulic cylinder 365. The primary hydraulic cylinder 365 has a primary cross-sectional area measured normal to the axis of movement of the housing 360. The hydraulic fluid pressure acts equally in all directions within the primary hydraulic cylinder 365 such that the hydraulic system applies a multiplying effect to the pneumatic force according to the ratio of the primary cross-sectional area : the secondary cross-sectional area to generate the output force of the hydraulic system. The output force urges the housing 360 in the first axial direction.

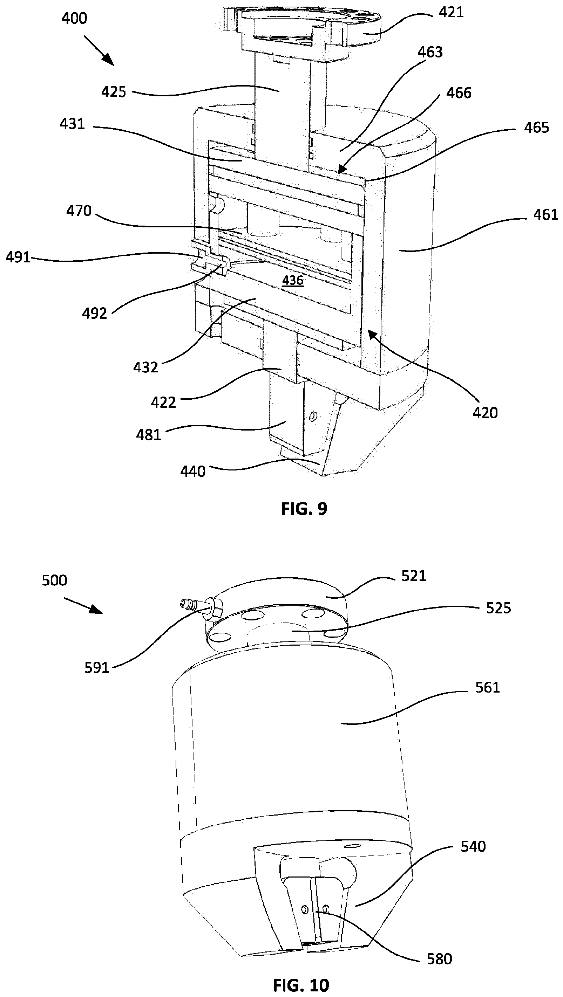

Referring now to Figures 8 and 9, there is shown another example grip assembly 400 for holding a sample for testing in a material testing machine. Where the features are the same as a previous example, the reference numerals are the same, other than the initial digit is "4". The assembly includes a core member 420, including a pneumatic cylinder 434 and pneumatic piston head 471, as well as a housing 460 substantially the same as the example described with reference to Figures 2 to 6.

The grip assembly 400 includes a conduit configured to fluidly connect a first port 491, connectable to a mains supply of compressed air, to a second port 492 that opens into the pneumatic cylinder 434.

The first port 491 is arranged on the core member 420 and aligned with an opening 469 extending through the sleeve 461 mounted around the core member 420. The opening 469 is configured so that the first port 491 remains accessible through the opening 469 as the housing 460 moves relative to the core member 420.

In the example, the opening 491 is an elongate opening with length greater than the range of axial travel between the housing 460 and the core member 420. Thus, in use, with the housing 460 in the first position, as shown in Figure 8, the first port 491 extends through the sleeve 460 proximal a first end 469a of the opening 469. As the housing 460 moves relative to the core member 420 in the first axial direction, indicated by the arrow B in Figure 8, then the sleeve 461 moves so that a second end 469b of the opening 469 approaches the first port 491 without contacting the first port 491. Thus, the housing 460 moves to its second position, urging the driving member 440 against the sample holder 480, without interference with the first port 491. The mains supply of compressed air is maintained as the housing 460 moves relative to the core member 420.

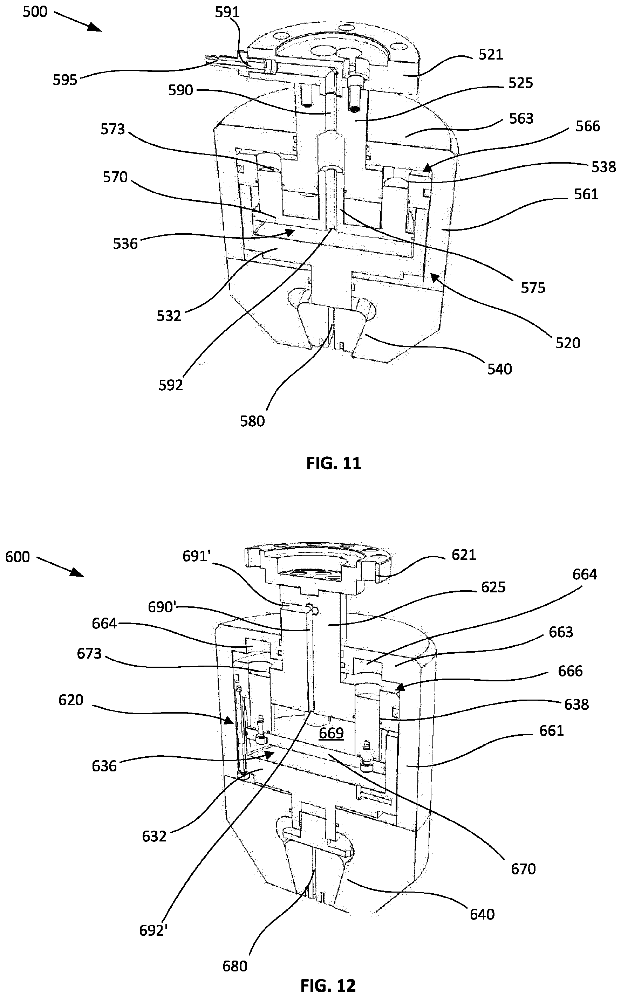

Referring now to Figures 10 and 11, there is shown another example grip assembly 500 for holding a sample for testing in a material testing machine. Where the features are the same as a previous example, the reference numerals are the same, other than the initial digit is "5". The grip assembly 500 includes a core member 520, including a pneumatic cylinder 534 and pneumatic piston head 571, as well as a housing 560 substantially the same as the example described with reference to Figures 2 to 6.

The grip assembly 500 includes a hydraulic system substantially the same as the example described with reference to Figures 2 to 6 such that the head member 570 includes six hydraulic piston heads 573 extending from the head member 570 in a direction of the first end portion 521. Each hydraulic piston head 573 is slidably received within a respective secondary hydraulic cylinder 538 formed through the first body element 531. Each secondary hydraulic cylinder 538 is arranged so that an open end of the secondary hydraulic cylinder 538 is fluidly connected to the primary hydraulic cylinder 565. That is, each secondary hydraulic volume 539 is fluidly connected to the primary hydraulic volume 566.

The grip assembly 500 includes a conduit 590 configured to fluidly connect a first port 591, connectable to a mains supply of compressed air, to a second port 592 that opens into the pneumatic cylinder 534. The first port 591 is provided on the core member 520 at the first end portion 521. A connector 595 is mounted within the first port 591 to enable the first port 591 to connect to an airline of the mains supply of compressed air.

The conduit 590 extends along the central axis A of the core member 520 to the second port 592. In particular, the conduit 590 extends axially through the first shaft 525 of the core member 520 from the first end portion 521 to the first body element 531.

The pneumatic piston head 571 is provided on the head member 570, moveable relative to the core member 520. The head member 570 includes a tube 575 arranged to extend through the head member 570 from the second port 592 towards the first end portion 521 so as to telescopingly engage with the conduit 590. That is, the tube 575 is slidably received within a portion of the conduit 590. In this way, the second port 592 is provided on the head member 570 and is fluidly connected to the conduit 590 in a compact and convenient arrangement. The conduit 590 remains fluidly connected to the pneumatic cylinder 534 as head member 570 moves relative to the conduit 590.

In the example, each of the six secondary hydraulic cylinders 538 has a cross-sectional area of 201mm$^2$ providing a secondary cross-sectional area of 1206mm$^2$. The primary cross-sectional area is the same as the example described with reference to Figures 2 to 6

In the example, the primary cross-sectional area is 9313mm$^2$. Thus a ratio of the primary cross-sectional area : the secondary cross-sectional area is

$$\frac{9313}{1206} = 7.72$$

The ratio of the primary cross-sectional area : the secondary cross-sectional area is a multiplying factor to the pneumatic force applied to the pneumatic piston head 571 in order to generate the output force of the hydraulic system. In the example grip assembly 500 the multiplying factor is 7.72. The pneumatic force applied to the pneumatic piston head 571 in the pneumatic cylinder 534 is multiplied by a factor of 7.72 to generate an output force for urging the driving member 540 against the sample holder 580 to apply the holding force.

Compressed air at 6 bar pressure supplied to the pneumatic cylinder 534 of the grip assembly 500 generates a pneumatic force of 4.6kN. The pneumatic force of the example is reduced compared to the example of Figures 2 to 6 because the second port 592 is provided through the pneumatic piston head 571. In this way, the pneumatic piston head area does not include the cross-sectional area of the second port 592. The pneumatic piston head area is thereby reduced by 201mm$^2$ to 7653mm$^2$, resulting in a pneumatic force of 4.6kN. The pneumatic force is multiplied by a factor of 7.72 to an output force of 35.4kN for urging the driving member 540.

Referring now to Figure 12, there is shown a further example grip assembly 600 for holding a sample for testing in a material testing machine. Where the features are the same as a previous example, the reference numerals are the same, other than the initial digit is "6". The grip assembly 600 includes a core member 620, including a pneumatic cylinder 634 and pneumatic piston head 671, as well as a housing 660 substantially the same as the example described with reference to Figures 2 to 6.

The grip assembly includes a biasing means including a pneumatic release chamber 669. The pneumatic release chamber 669 is provided to in a volume defined between the first body element and the head member. In this way, the six hydraulic piston heads 673 the extend from the head member 670 through the pneumatic release chamber 669. The hydraulic piston heads 673 extend through the pneumatic release chamber 669 in a direction of the first end portion 621.

The grip assembly 600 includes a first conduit (not shown) configured to fluidly connect a first port, connectable to a mains supply of compressed air, to a second port that opens into the pneumatic cylinder 634. Optionally, a connector is mounted within the first port to enable the first port to connect to an air-line of the mains supply of compressed air.

The grip assembly 600 also includes a second conduit 690' configured to fluidly connect a first release port 691', connectable to a mains supply of compressed air, to a second release port 692' that opens into pneumatic release chamber 669. The first release port 691' is provided on the core member 620 at the first end portion 621. Optionally, a connector is mounted within the first release port 691' to enable the first release port 691' to connect to a further air-line of the mains supply of compressed air.

The second conduit 690 extends along the central axis A of the core member 620 to the second release port 692'. In particular, the second conduit 690' extends axially through the first shaft 625 of the core member 620 from the first end portion 621 to the first body element 631.

In use, after testing of a sample is completed the pneumatic release chamber 669 is used to remove the sample when the pneumatic pressure, providing the holding force, is released from the pneumatic cylinder 636. Compressed air is selectively supplied to the second release port 692' of the pneumatic release chamber 669 via the second conduit 690'. A pneumatic release pressure is thereby provided to the pneumatic release chamber 669 to move the head member 670 towards its first position. The pneumatic release pressure in the pneumatic release chamber 669 moves the head member 670 towards the second end portion, thereby overcoming any inertia or sticking between components and ensure that the housing moves in the second axial direction. Optionally, the biasing means may also include suitable springs to move the housing in the second axial direction.

In the example shown in Figure 12, the hydraulic system is substantially the same as the example described with reference to Figures 2 to 6. The primary hydraulic cylinder 665 includes a series of recesses 664 provided on the inner surface of the base 663 of the housing 660. Each recess 664 is axially aligned with a respective secondary hydraulic cylinder 638.

Each hydraulic piston head 673 is slidably received within a respective secondary hydraulic cylinder 638 formed through the first body element. When moving in the first axial direction, each hydraulic piston head 673 moves through the secondary hydraulic cylinder 638 towards a respective recess 664. By providing a series of recesses 664 to receive each hydraulic piston head 673 an even more compact grip assembly is provided.

The primary hydraulic cylinder 665 has a primary cross-sectional area. That is, the primary hydraulic cylinder 665 has a primary cross-sectional area measured normal to the axis of movement of the housing 660. In the example, the primary cross-sectional area is the total cross-sectional areas of both the inner surface of the base 663 and closed ends of the series of recesses 664 provided therein. In this way, the primary cross-sectional area is the same as if recesses were not provided in the inner surface of the base 663. In the example, the primary cross-sectional area and the secondary cross-sectional area are the same as those provided for the example of Figures 2 to 6. The multiplying factor applied to the pneumatic force on the pneumatic piston head to generate the output force is also the same.

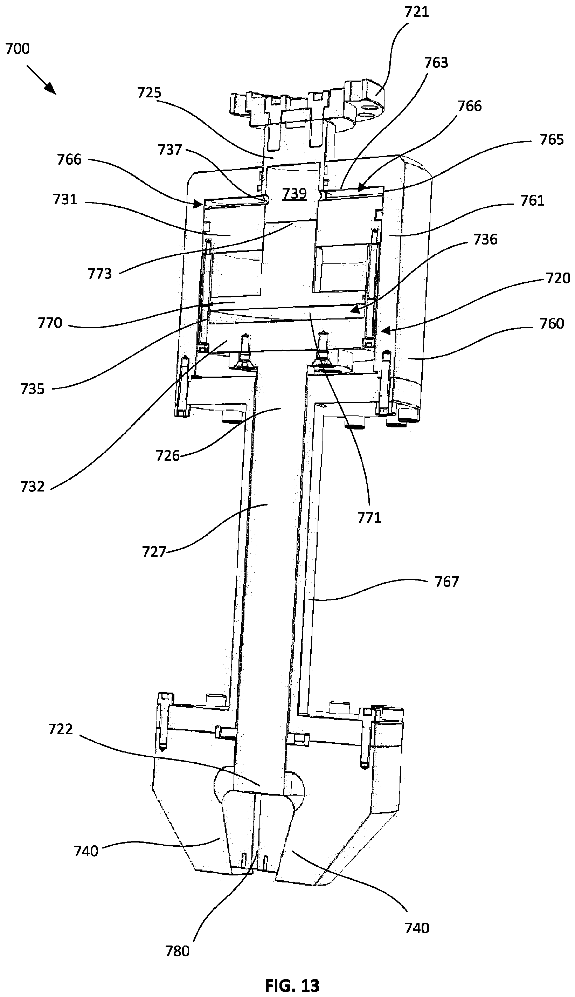

Referring now to Figure 13, there is shown another example grip assembly 700 for holding a sample for testing in a material testing machine. Where the features are the same as a previous example, the reference numerals are the same, other than the initial digit is "7". The grip assembly 700 includes a core member 720, including a pneumatic cylinder 734 and pneumatic piston head 771, and a housing 760 substantially the same as the example described with reference to Figure 7.

The core member 720 has a second shaft 726 including a spacer element 727. The spacer element 727 is provided between the body portion of the core member 720 and the second end portion 722 to provide spatial separation of the sample holder 780 from the housing 760.

The housing includes a secondary sleeve 767 coaxially mounted around the spacer element 727. The secondary sleeve 767 extends from the sleeve 761 to the driving member 740. As the housing 760 moves relative to the core member then the secondary sleeve 767 moves relative to the spacer element 727, thereby urging the driving member 740 against the sample holder 780 in the manner described with respect to the other examples disclosed herein.

By providing a spacer element 727 and secondary sleeve 767, the sample holder 780 and driving member 740 may be provided in an environmental chamber, for example an oven, without requiring the remainder of the grip assembly 700 to be within the environmental chamber.

The grip assembly 700 includes a hydraulic system substantially the same as the example described with reference to Figure 7 such that the head member 770 includes a hydraulic piston head 773 extending from the head member 770 in a direction of the first end portion 721. The hydraulic piston head 773 is disposed axially within the core member and is slidably received within a secondary hydraulic cylinder, enclosing a secondary hydraulic volume 739, formed axially through the first body element 731.

The secondary hydraulic cylinder is arranged so that an open end of the secondary hydraulic cylinder is fluidly connected to the primary hydraulic cylinder 765 of the housing 760 through openings 737 in the wall of the secondary hydraulic cylinder.

In use, an elevated pneumatic pressure is provided in the pneumatic cylinder 735 using supplied compressed air, using an example conduit and first and second ports as described herein. The housing 760, including the secondary sleeve 767, thereby moves from a first position towards the first end portion 721. The housing 760 moves in the first axial direction thereby urging the driving member 740 against the sample holder 780 to provide the holding force.

In the example, the primary cross-sectional area and the secondary cross-sectional area are the same as those provided for the example of Figure 7. The multiplying factor applied to the pneumatic force on the pneumatic piston head to generate the output force is also the same.

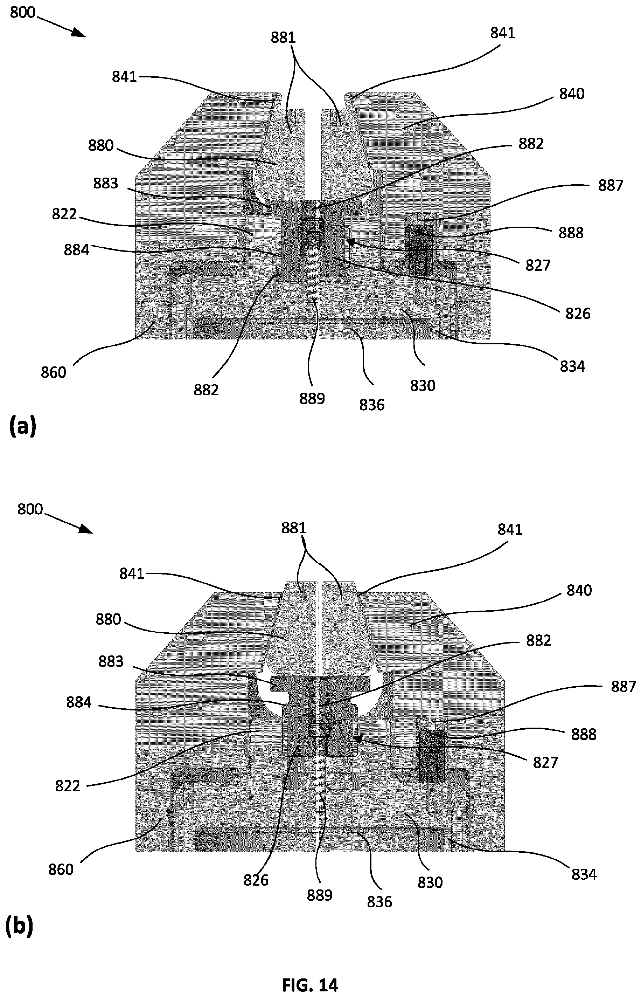

Referring now Figure 14(a) and 14(b), there are shown a portion of another example grip assembly 800 for holding a sample for testing in a material testing machine. Where the features are the same as a previous example, the reference numerals are the same, other than the initial digit is "8". The grip assembly 800 includes a core member 820, including a pneumatic cylinder 834 a pneumatic piston head 871, and a housing 860 substantially the same as the example described with reference to other Figures, The grip assembly 800 includes a preset spacing between the pair of jaws 881 prior to applying a holding force to the sample, wherein the pre-set spacing is selectively adjustable. The grip assembly 800 is adjustable between a maximum pre-set spacing (Fig. 14(a)) and a minimum pre-set spacing (Fig. 14(b)) according the thickness of the sample undergoing testing.

The core member 820 has a spacer element 827. In the example, the spacer element 827 includes a second shaft 826 that extends from a first end proximal to the body portion 830 to a second end proximal to the sample holder 880. The spacer element 827 includes an outer cylindrical surface 884, as well as bore 882 extending axially through the second shaft 826.

The spacer element 827 is arranged at the second end portion 822 to provide an axial separation between the sample holder 880 and the body portion 830. In particular, the spacer element 827 is configured to provide an adjustable axial separation between the sample holder 880 and the body portion 830 of the core member 820. In this way, a selectively adjustable pre-set spacing between the pair of jaws 881 is provided.

The spacer element 827 is coaxially mounted to the second end portion 822 to be rotatable relative to the core member 820 around the central axis of the core member 820. The spacer element 827 is movably mounted to the body portion 830 so as to allow relative axial movement of the spacer element 827 relative to the body portion.

In the example, the spacer element 827 is mounted to the second end portion 822 using a fastening bolt 889 inserted through the bore 882 to be received and fixed to the body portion 830. The fastening bolt 889 is configured to fix to the body portion 830 while allowing axial travel of the spacer element 827 along the fastening bolt 889.

The outer cylindrical surface 884 of the spacer element 827 includes a screw thread that is operable engaged with a mating thread provided on the second end portion 822. In this way, the second shaft 826 may be rotated relative to the core member 820 prior to use, causing the second shaft 826 to be urged in an axial direction, either towards or away from the core member 820.

The spacer element 827 includes a first stop element 882. The first stop element 882 is located on the outer cylindrical surface 884, distal to the sample holder 880. The first stop element 882 limits the axial separation between the sample holder 880 and the body portion 830 of the core member 820 to a maximal axial separation.

The spacer element 827 includes a second stop element 883. The second stop element 883 is located on the outer cylindrical surface 884, proximal to the sample holder 880. The second stop element 883 limits the axial separation between the sample holder and the body portion of the core member to a minimal axial separation.

In use, the grip assembly 800 is operated in substantially the same way as other examples described here, such as the example described with reference to Fig. 12, except that the pre-set spacing between the pair of jaws 881 is adjustable prior to activating the grip assembly 800 to apply a holding force.

For example, to accommodate samples with increased thickness for testing, the pre-set spacing between the pair of jaws 881 is adjusted from a first pre-set spacing to a wider, second pre-set spacing. The user rotates the spacer element 827 relative to the second end portion 822 in a first direction, drawing the sample holder 880, including the pair of jaws 881, towards the core member 830. The effective axial length of the spacer 827 is thereby reduced. The sample holder 880 is thus moved axially relative to the mutually-opposed angled engaging faces 841 of the driving member 840. The sample holder 880 moves from a first axial position to a second axial position, wherein the second axial position is closer to the core member 830. In the second axial position, the sample holder 880 engages portions of the engaging faces 841 at a point where the portions are set further apart than at the first axial position. The pre-set spacing between the pair of jaws 881 is correspondingly increased.

For example, to accommodate samples with reduced thickness for testing, the pre-set spacing between the pair of jaws 881 is adjusted from a third pre-set spacing to a narrower, fourth pre-set spacing. The user rotates the spacer element 827 relative to the core member 830 in an opposing, second direction, urging the sample holder 880, including the pair of jaws 881, away from the core member 830. The effective axial length of the spacer 827 is thereby increased. The sample holder 880 is thus moved axially relative to the mutually-opposed angled engaging faces 841 of the driving member 840. The sample holder moves from a third axial position to a fourth axial position, wherein the fourth axial position is further from the core member 830. In the fourth axial position, the sample holder 880 engages portions of the engaging faces 841 at a point where the portions are set closer together than at the third axial position. The pre-set spacing between the pair of jaws 881 is correspondingly reduced.

The housing 860 is mounted coaxially around the body portion 830 and configured to prevent relative rotational movement between the housing 860 and the body portion 830. In the example shown, the body portion 830 has a post 888. The post 888 projects from the body portion 830 towards the driving member 840. The post 888 is received within a recess 887 on an inner surface of the housing 860. The recess 887 extends into the housing in a direction parallel to the central axis. In this way, as the housing 860 moves relative to the core member, the post is free to move in an axial direction within the recess 887. However, the post 888 is rotationally constrained within the recess 887, preventing relative rotation between the housing 860 and the core member 830.

In the example, the primary cross-sectional area and the secondary cross-sectional area are the same as those provided for the example of Figure 12. The multiplying factor applied to the pneumatic force on the pneumatic piston head to generate the output force is also the same.

As will be appreciated, the adjustable pre-set spacing of the pair of jaws may be adapted for use in any of the grip assemblies described here. Likewise, the means for preventing relative rotational movement between housing and body portion may also be adapted for any of the grip assemblies.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. For example, each of the disclosed arrangements of ports and conduit may be compatible with core member, housing and head member arrangement disclosed herein. The invention is defined by the appended claims.

## Claims

1. A grip assembly (200) for holding a sample for testing in a material testing machine, comprising:

    a core member (220) extending along a central axis from a first end portion (221), configured to releasably mount the grip assembly to a crosshead (108), to a second end portion (222), wherein the core member (220) comprises a body portion (230) having a pneumatic cylinder (234) enclosing a pneumatic volume (236);
    a pneumatic piston head (271) arranged to be slidably received in the pneumatic cylinder (234) and to be moveable along the pneumatic cylinder (234) in a first direction to expand the pneumatic volume (236), and in a second, opposing, direction to contract the pneumatic volume (236);

    a housing (260), including a sleeve member (262) coaxially mounted around the body portion (230) so that the housing (260) is moveable relative to the core member (220) in a first axial direction towards the first end portion (221) and in a second, opposing axial direction;
    a sample holder (280) disposed at the second end portion (222), configured to selectively apply a holding force to a sample for testing; and
    a driving member (240) configured to contactingly engage the sample holder (280);
    wherein the grip assembly (200) is configured so that, in response to the pneumatic piston head (271) moving in the first direction, the housing (260) moves in the first axial direction thereby urging the driving member (240) against the sample holder (280) to provide the holding force.

2. A grip assembly according to claim 1, wherein the pneumatic cylinder is oriented to be coaxial with the central axis of the core member so that the first direction of movement of the pneumatic piston head is in the first axial direction, and the second direction of movement is in the second axial direction.

3. A grip assembly according to any preceding claim, wherein the pneumatic piston head (271) is provided on a head member (270) configured to move relative to the core member (220) in the first direction and the second direction.

4. A grip assembly according to any of claims 1 to 3, further comprising a hydraulic system having a primary hydraulic cylinder (256) formed in the housing (260), wherein the primary hydraulic cylinder (256) encloses a primary hydraulic volume (266), and wherein the housing (260) is configured to engage with the body portion (230) so that the primary hydraulic volume (266) expands when housing (260) moves relative to the core member (220) in the first axial direction and contracts when the housing (260) moves relative to the core member (220) in the second, opposing, axial direction.

5. A grip assembly according to claim 4, wherein the primary hydraulic cylinder has a primary cross-sectional area, preferably defined by a base (263) of the housing.

6. A grip assembly according to claim 4 or claim 5, wherein the primary hydraulic cylinder is oriented to be coaxial with the central axis of the core member.

7. A grip assembly according to any of claims 4 to 6, wherein the hydraulic system further comprises at least one secondary hydraulic cylinder (238), enclosing a secondary hydraulic volume (239) that is fluidly connected to the primary hydraulic volume (266), wherein a hydraulic piston head (275) is arranged to be slidably received in each of the at least one secondary hydraulic cylinders (238) and to be moveable therealong in a third direction to contract the secondary hydraulic volume (239), and in a fourth, opposing, direction to expand the secondary hydraulic volume (239).

8. A grip assembly according to claim 7, wherein as the pneumatic piston head moves in the first direction, each hydraulic piston head moves in the third direction thereby contracting the secondary hydraulic volume and expanding the primary hydraulic volume.

9. A grip assembly according to claim 7 or claim 8, wherein the hydraulic piston heads are configured to move in the first direction a distance in a range from 5mm to 60mm and, preferably, in a range from 20mm to 50mm.

10. A grip assembly according to any of claims 7 to 9, wherein each of the at least one secondary hydraulic cylinder is formed through the body portion.

11. A grip assembly according to any of claims 7 to 10, wherein the head member has the at least one hydraulic piston head mounted thereto, preferably releasably mounted thereto, so that the at least one hydraulic piston head is arranged to project from the head member a direction towards the first end portion of the core member.

12. A grip assembly according any of claims 7 to 11, wherein the at least one secondary hydraulic cylinder comprises a plurality of secondary hydraulic cylinders, preferably from 2 to 8 secondary hydraulic cylinders and, more preferably, from 3 to 6 secondary hydraulic cylinders.

13. A grip assembly according to any of claims 7 to 12, wherein each of the at least one secondary hydraulic cylinders includes a secondary central axis, and wherein each secondary central axis is arranged parallel to the central axis of the core member and, preferably, the grip assembly includes at least two secondary hydraulic cylinders wherein the respective secondary central axes are arranged circumferentially around the central axis of the core member.

14. A grip assembly according to any of claims 7 to 13, wherein the at least one secondary hydraulic cylinders cumulatively provide a secondary cross-sectional area, and wherein a ratio of the primary cross-sectional area : the secondary cross-sectional area is in a range of from 1:2 to 1:20 and, preferably, in a range of from 1:5 to 1:10.

15. A grip assembly according to any preceding claim, wherein the sample holder is mounted to the second end portion of the core member and the driving member extends from the housing to contactingly engage the sample holder.

16. A grip assembly according to preceding claim, wherein the sample holder is mounted to the driving member and is configured to abut the second end portion so that the second end portion prevents the sample holder moving with the driving member in first axial direction.

17. A grip assembly according to claim 15 or claim 16, wherein the sample holder comprises a mutually-opposed pair of jaws (281), wherein at least one of the pair of jaws is configured to slidably move in a direction transverse to the central axis.

18. A grip assembly according to claim 16, wherein the sample holder comprises a pre-set spacing between the pair of jaws prior to applying the holding force to the sample and, preferably, wherein the pre-set spacing is selectively adjustable.

19. A grip assembly according to any preceding claim, wherein the driving member includes a mutually-opposed pair of engaging faces (241), preferably wherein each engaging face (241) is angled relative to the central axis in a range from 10° to 45°, and wherein each engaging face (241) is configured to contactingly engage the sample holder and thereby urge the sample holder to provide the holding force.

20. A grip assembly according to any preceding claim, wherein the core member includes a spacer element (727) disposed between the second end portion and the body portion.

21. A grip assembly according to any preceding claim, wherein the housing is configured to move relative to the core member in first axial direction for a distance in a range of from 1mm to 10mm and, preferably, in a range of from 2mm to 7mm.

22. A grip assembly according to any preceding claim, further comprising a biasing means configured to move the housing relative to the core member in a direction towards the second end portion.

23. A grip assembly according to any preceding claim, further comprising a conduit (590) configured to fluidly connect a first port (591), connectable to a mains supply of compressed air, to a second port (592) that opens into the pneumatic cylinder.

24. A grip assembly according to claim 23, wherein the first port is provided on the core member at, or proximal to, the first end portion, and wherein the conduit extends along the central axis of the core member to the second port and, optionally, wherein the pneumatic piston head is provided on a head member, moveable relative to the core member, and wherein the head member includes a tube arranged to extend through the head member from the second port towards the first end portion so as to telescopingly engage with the conduit.

25. A grip assembly according to claim 23, wherein the first port is arranged on the core member and aligned with an opening extending through the sleeve mounted around the core member, and wherein the opening is configured so that the first port remains accessible through the opening as the housing moves relative to the core member.

**Patentansprüche**

1. Griffanordnung (200) zum Halten einer Probe zum Testen in einer Materialprüfmaschine, aufweisend:

    ein Kernelement (220), das sich entlang einer zentralen Achse von einem ersten Endabschnitt (221), der so eingerichtet ist, dass er die Griffanordnung lösbar an einem Kreuzkopf (108) befestigt, zu einem zweiten Endabschnitt (222) erstreckt, wobei das Kernelement (220) einen Körperabschnitt (230) aufweist, der einen pneumatischen Zylinder (234) aufweist, der ein pneumatisches Volumen (236) umschließt;
    einen pneumatischen Kolbenboden (271), der so angeordnet ist, dass er verschiebbar in dem pneumatischen Zylinder (234) aufgenommen und entlang dem pneumatischen Zylinder (234) in einer ersten Richtung bewegbar ist, um das pneumatische Volumen (236) auszudehnen, und in einer zweiten, entgegengesetzten Richtung, um das pneumatische Volumen (236) zu kontrahieren;
    ein Gehäuse (260), das ein Hülsenelement (262) umfasst, das koaxial um den Körperabschnitt (230) befestigt ist, sodass das Gehäuse (260) relativ zu dem Kernelement (220) in einer ersten axialen Richtung in Richtung des ersten Endabschnitts (221) und in einer zweiten, entgegengesetzten axialen Richtung bewegbar ist;
    einen Probenhalter (280), der an dem zweiten Endabschnitt (222) angeordnet und so eingerichtet ist, dass er selektiv eine Haltekraft auf eine Probe zum Testen ausübt; und
    ein Antriebselement (240), das so eingerichtet ist, dass es mit dem Probenhalter (280) in Kontakt eingreift;
    wobei die Griffanordnung (200) so eingerichtet ist, dass sich das Gehäuse (260) in Reaktion auf die Bewegung des pneumatischen Kolbenbodens (271) in der ersten Richtung in der ersten axialen Richtung bewegt, wodurch das Antriebselement (240) gegen den Probenhalter (280) gedrückt wird, um die Haltekraft bereitzustellen.

2. Griffanordnung nach Anspruch 1, wobei der pneumatische Zylinder so ausgerichtet ist, dass er koaxial zu der zentralen Achse des Kernelements liegt, sodass die erste Bewegungsrichtung des pneumatischen Kolbenbodens in der ersten axialen Richtung liegt und die zweite Bewegungsrichtung in der zweiten axialen Richtung liegt.

3. Griffanordnung nach einem der vorhergehenden Ansprüche, wobei der pneumatische Kolbenboden (271) an einem Bodenelement (270) vorgesehen ist, das so eingerichtet ist, dass es sich relativ zu dem Kernelement (220) in der ersten Richtung und der zweiten Richtung bewegt.

4. Griffanordnung nach einem der Ansprüche 1 bis 3, die ferner ein Hydrauliksystem mit einem primären Hydraulikzylinder (256), der in dem Gehäuse (260) ausgebildet ist, aufweist, wobei der primäre Hydraulikzylinder (256) ein primäres Hydraulikvolumen (266) einschließt, und wobei das Gehäuse (260) so eingerichtet ist, dass es mit dem Körperabschnitt (230) in Eingriff steht, sodass sich das primäre Hydraulikvolumen (266) ausdehnt, wenn sich das Gehäuse (260) relativ zu dem Kernelement (220) in der ersten axialen Richtung bewegt, und sich kontrahiert, wenn sich das Gehäuse (260) relativ zu dem Kernelement (220) in der zweiten, entgegengesetzten axialen Richtung bewegt.

5. Griffanordnung nach Anspruch 4, wobei der primäre Hydraulikzylinder eine primäre Querschnittsfläche aufweist, die vorzugsweise durch eine Basis (263) des Gehäuses definiert ist.

6. Griffanordnung nach Anspruch 4 oder Anspruch 5, wobei der primäre Hydraulikzylinder so ausgerichtet ist, dass er koaxial zu der zentralen Achse des Kernelements liegt.

7. Griffanordnung nach einem der Ansprüche 4 bis 6, wobei das Hydrauliksystem ferner mindestens einen sekundären Hydraulikzylinder (238) aufweist, der ein sekundäres Hydraulikvolumen (239) einschließt, das mit dem primären Hydraulikvolumen (266) fluidisch verbunden ist, wobei ein hydraulischer Kolbenboden (275) so angeordnet ist, dass er in jedem aus dem mindestens einen sekundären Hydraulikzylinder (238) verschiebbar aufgenommen und entlang diesem in einer dritten Richtung bewegbar ist, um das sekundäre Hydraulikvolumen (239) zu kontrahieren, und in einer vierten entgegengesetzten Richtung, um das sekundäre Hydraulikvolumen (239) auszudehnen.

8. Griffanordnung nach Anspruch 7, wobei, wenn sich der pneumatische Kolbenboden in der ersten Richtung bewegt, sich jeder hydraulische Kolbenboden in der dritten Richtung bewegt, wodurch das sekundäre Hydraulikvolumen kontrahiert und das primäre Hydraulikvolumen ausgedehnt wird.

9. Griffanordnung nach Anspruch 7 oder Anspruch 8, wobei die hydraulischen Kolbenböden so eingerichtet sind, dass sie sich in der ersten Richtung um einen Abstand in einem Bereich von 5 mm bis 60 mm und vorzugsweise in einem Bereich von 20 mm bis 50 mm bewegen.

10. Griffanordnung nach einem der Ansprüche 7 bis 9, wobei jeder aus dem mindestens einen sekundären Hydraulikzylinder durch den Körperabschnitt hindurch ausgebildet ist.

11. Griffanordnung nach einem der Ansprüche 7 bis 10, wobei an dem Bodenelement der mindestens eine Hydraulikkolbenboden befestigt ist, vorzugsweise lösbar daran befestigt ist, sodass der mindestens eine Hydraulikkolbenboden so angeordnet ist, dass er von dem Bodenelement in Richtung des ersten Endabschnitts des Kernelements vorsteht.

12. Griffanordnung nach einem der Ansprüche 7 bis 11, wobei der mindestens eine sekundäre Hydraulikzylinder mehrere sekundäre Hydraulikzylinder aufweist, vorzugsweise zwei bis acht sekundäre Hydraulikzylinder und insbesondere drei bis sechs sekundäre Hydraulikzylinder.

13. Griffanordnung nach einem der Ansprüche 7 bis 12, wobei jeder aus dem mindestens einen sekundären Hydraulikzylinder eine sekundäre zentrale Achse aufweist, und wobei jede sekundäre zentrale Achse parallel zu der zentralen Achse des Kernelements angeordnet ist und vorzugsweise die Griffanordnung mindestens zwei sekundäre Hydraulikzylinder umfasst, wobei die jeweiligen sekundären zentralen Achsen in Umfangsrichtung um die zentrale Achse des Kernelements angeordnet sind.

14. Griffanordnung nach einem der Ansprüche 7 bis 13, wobei die mindestens einen sekundären Hydraulikzylinder kumulativ eine sekundäre Querschnittsfläche bereitstellen, und wobei ein Verhältnis der primären Querschnittsfläche zu der sekundären Querschnittsfläche in einem Bereich von 1:2 bis 1:20 und vorzugsweise in einem Bereich von 1:5 bis 1:10 liegt.

15. Griffanordnung nach einem der vorhergehenden Ansprüche, wobei der Probenhalter an dem zweiten Endabschnitt des Kernelements befestigt ist und sich das Antriebselement von dem Gehäuse erstreckt, um mit dem Probenhalter in Kontakt einzugreifen.

16. Griffanordnung nach einem der vorangehenden Ansprüche, wobei der Probenhalter an dem Antriebselement befestigt und so eingerichtet ist, dass er an dem zweiten Endabschnitt anliegt, sodass der zweite Endabschnitt verhindert, dass sich der Probenhalter mit dem Antriebselement in einer ersten axialen Richtung bewegt.

17. Griffanordnung nach Anspruch 15 oder Anspruch 16, wobei der Probenhalter ein einander gegenüberliegendes Paar von Backen (281) aufweist, wobei mindestens eine aus dem Paar von Backen so eingerichtet ist, dass sie sich in einer Richtung quer zu der zentralen Achse verschiebbar bewegt.

18. Griffanordnung nach Anspruch 16, wobei der Probenhalter einen vorbestimmten Abstand zwischen dem Paar von Backen aufweist, bevor die Haltekraft auf die Probe aufgebracht wird, und wobei vorzugsweise der vorbestimmte Abstand selektiv einstellbar ist.

19. Griffanordnung nach einem der vorhergehenden Ansprüche, wobei das Antriebselement ein einander gegenüberliegendes Paar von Eingriffsflächen (241) umfasst, wobei vorzugsweise jede Eingriffsfläche (241) relativ zu der zentralen Achse in einem Bereich von 10° bis 45° abgewinkelt ist, und wobei jede Eingriffsfläche (241) so eingerichtet ist, dass sie mit dem Probenhalter in Kontakt eingreift und dadurch den Probenhalter so drückt, dass er die Haltekraft bereitstellt.

20. Griffanordnung nach einem der vorhergehenden Ansprüche, wobei das Kernelement ein Abstandselement (727) aufweist, das zwischen dem zweiten Endabschnitt und dem Körperabschnitt angeordnet ist.

21. Griffanordnung nach einem der vorhergehenden Ansprüche, bei der das Gehäuse so eingerichtet ist, dass es sich relativ zu dem Kernelement in einer ersten axialen Richtung über einen Abstand in einem Bereich von 1 mm bis 10 mm und vorzugsweise in einem Bereich von 2 mm bis 7 mm bewegt.

22. Griffanordnung nach einem der vorhergehenden Ansprüche, die ferner ein Vorspannmittel aufweist, das so eingerichtet ist, dass es das Gehäuse relativ zu dem Kernelement in einer Richtung zu dem zweiten Endabschnitt bewegt.

**23.** Griffanordnung nach einem der vorhergehenden Ansprüche, die ferner eine Leitung (590) aufweist, die so eingerichtet ist, dass sie einen ersten Anschluss (591), der mit einer Drucklufthauptversorgung verbindbar ist, mit einem zweiten Anschluss (592), der sich in den pneumatischen Zylinder öffnet, fluidisch verbindet.

**24.** Griffanordnung nach Anspruch 23, wobei der erste Anschluss an dem Kernelement an oder proximal zu dem ersten Endabschnitt vorgesehen ist, und wobei sich die Leitung entlang der zentralen Achse des Kernelements zu dem zweiten Anschluss erstreckt und wobei optional der pneumatische Kolbenboden an einem Bodenelement vorgesehen ist, das relativ zu dem Kernelement beweglich ist, und wobei das Bodenelement eine Röhre beinhaltet, die so angeordnet ist, dass sie sich durch das Bodenelement von dem zweiten Anschluss in Richtung des ersten Endabschnitts erstreckt, um teleskopartig in die Leitung einzugreifen.

**25.** Griffanordnung nach Anspruch 23, wobei der erste Anschluss an dem Kernelement angeordnet ist und an einer Öffnung ausgerichtet ist, die sich durch die Hülse erstreckt, die um das Kernelement herum befestigt ist, und wobei die Öffnung so eingerichtet ist, dass der erste Anschluss durch die Öffnung zugänglich bleibt, wenn sich das Gehäuse relativ zu dem Kernelement bewegt.

**Revendications**

**1.** Ensemble de préhension (200) destiné à maintenir un échantillon à mettre à l'essai dans une machine d'essai de matériau, comprenant:

un organe noyau (220) s'étendant le long d'un axe central depuis une première portion d'extrémité (221), configurée pour monter de manière libérable l'ensemble de préhension sur une traverse (108), jusqu'à une deuxième portion d'extrémité (222), dans lequel l'organe noyau (220) comprend une portion de corps (230) ayant un vérin pneumatique (234) renfermant un volume pneumatique (236);
une tête de piston pneumatique (271) agencée pour être reçue de manière coulissante dans le vérin pneumatique (234) et pour être mobile le long du vérin pneumatique (234) dans une première direction pour dilater le volume pneumatique (236), et dans une deuxième direction opposée pour contracter le volume pneumatique (236);
un logement (260), comprenant un organe manchon (262) monté coaxialement autour de la portion de corps (230) de sorte que le logement (260) est mobile par rapport à l'organe noyau (220) dans une première direction axiale vers la première portion d'extrémité (221) et dans une deuxième direction axiale opposée;
un porte-échantillon (280) disposé au niveau de la deuxième portion d'extrémité (222), configuré pour appliquer sélectivement une force de maintien à un échantillon à mettre à l'essai; et
un organe d'entraînement (240) configuré pour venir en prise avec le porte-échantillon (280);
dans lequel l'ensemble de préhension (200) est configuré de telle sorte que, en réponse au déplacement de la tête de piston pneumatique (271) dans la première direction, le logement (260) se déplace dans la première direction axiale, poussant ainsi l'organe d'entraînement (240) contre le porte-échantillon (280) pour fournir la force de maintien.

**2.** Ensemble de préhension selon la revendication 1, dans lequel le vérin pneumatique est orienté de manière à être coaxial avec l'axe central de l'organe noyau de telle sorte que la première direction de déplacement de la tête de piston pneumatique est dans la première direction axiale, et la deuxième direction de déplacement est dans la deuxième direction axiale.

**3.** Ensemble de préhension selon l'une quelconque des revendications précédentes, dans lequel la tête de piston pneumatique (271) est prévue sur un organe de tête (270) configuré pour se déplacer par rapport à l'organe noyau (220) dans la première direction et la deuxième direction.

**4.** Ensemble de préhension selon l'une quelconque des revendications 1 à 3, comprenant en outre un système hydraulique ayant un vérin hydraulique primaire (256) formé dans le logement (260), dans lequel le vérin hydraulique primaire (256) renferme un volume hydraulique primaire (266), et dans lequel le logement (260) est configuré pour venir en prise avec la portion de corps (230) de sorte que le volume hydraulique primaire (266) se dilate lorsque le logement (260) se déplace par rapport à l'organe noyau (220) dans la première direction axiale et se contracte lorsque le logement (260) se déplace par rapport à l'organe noyau (220) dans la deuxième direction axiale opposée.

**5.** Ensemble de préhension selon la revendication 4, dans lequel le vérin hydraulique primaire a une surface de coupe transversale primaire, de préférence définie par une base (263) du logement.

**6.** Ensemble de préhension selon la revendication 4 ou la revendication 5, dans lequel le vérin hydraulique primaire est orienté de manière à être coaxial avec l'axe central de l'organe noyau.

7. Ensemble de préhension selon l'une quelconque des revendications 4 à 6, dans lequel le système hydraulique comprend en outre au moins un vérin hydraulique secondaire (238), enfermant un volume hydraulique secondaire (239) qui est raccordé fluidiquement au volume hydraulique primaire (266), dans lequel une tête de piston hydraulique (275) est agencée pour être reçue de manière coulissante dans chacun de l'au moins un vérin hydraulique secondaire (238) et pour être mobile le long de celui-ci dans une troisième direction afin de contracter le volume hydraulique secondaire (239), et dans une quatrième direction opposée pour dilater le volume hydraulique secondaire (239).

8. Ensemble de préhension selon la revendication 7, dans lequel, lorsque la tête de piston pneumatique se déplace dans la première direction, chaque tête de piston hydraulique se déplace dans la troisième direction, contractant ainsi le volume hydraulique secondaire et dilatant le volume hydraulique primaire.

9. Ensemble de préhension selon la revendication 7 ou la revendication 8, dans lequel les têtes de piston hydraulique sont configurées pour se déplacer dans la première direction sur une distance dans une plage de 5 mm à 60 mm et, de préférence, dans une plage de 20 mm à 50 mm.

10. Ensemble de préhension selon l'une quelconque des revendications 7 à 9, dans lequel chacun de l'au moins un vérin hydraulique secondaire est formé à travers la portion de corps.

11. Ensemble de préhension selon l'une quelconque des revendications 7 à 10, dans lequel l'organe de tête a l'au moins une tête de piston hydraulique montée sur celui-ci, de préférence montée de manière amovible sur celui-ci, de telle sorte que l'au moins une tête de piston hydraulique est agencée pour faire saillie depuis l'organe de tête dans une direction vers la première portion d'extrémité de l'organe noyau.

12. Ensemble de préhension selon l'une quelconque des revendications 7 à 11, dans lequel l'au moins un vérin hydraulique secondaire comprend une pluralité de vérins hydrauliques secondaires, de préférence de 2 à 8 vérins hydrauliques secondaires et, de manière davantage préférée, de 3 à 6 vérins hydrauliques secondaires.

13. Ensemble de préhension selon l'une quelconque des revendications 7 à 12, dans lequel chacun de l'au moins un vérin hydraulique secondaire comporte un axe central secondaire, et dans lequel chaque axe central secondaire est agencé parallèlement à l'axe central de l'organe noyau et, de préférence, l'ensemble de préhension comporte au moins deux vérins hydrauliques secondaires dans lequel les axes centraux secondaires respectifs sont agencés circonférentiellement autour de l'axe central de l'organe noyau.

14. Ensemble de préhension selon l'une quelconque des revendications 7 à 13, dans lequel l'au moins un vérin hydraulique secondaire fournit de manière cumulative une surface de coupe transversale secondaire, et dans lequel un rapport de la surface de coupe transversale primaire : la surface de coupe transversale secondaire est dans une plage de 1:2 à 1:20 et, de préférence, dans une plage de 1:5 à 1:10.

15. Ensemble de préhension selon l'une quelconque des revendications précédentes, dans lequel le porte-échantillon est monté sur la deuxième portion d'extrémité de l'organe noyau et l'organe d'entraînement s'étend à partir du logement pour venir en prise par contact avec le porte-échantillon.

16. Ensemble de préhension selon la revendication précédente, dans lequel le porte-échantillon est monté sur l'organe d'entraînement et est configuré pour buter contre la deuxième portion d'extrémité de telle sorte que la deuxième portion d'extrémité empêche le porte-échantillon de se déplacer avec l'organe d'entraînement dans la première direction axiale.

17. Ensemble de préhension selon la revendication 15 ou la revendication 16, dans lequel le porte-échantillon comprend une paire de mâchoires mutuellement opposées (281), dans lequel au moins l'une de la paire de mâchoires est configurée pour se déplacer de manière coulissante dans une direction transversale à l'axe central.

18. Ensemble de préhension selon la revendication 16, dans lequel le porte-échantillon comprend un espacement préréglé entre la paire de mâchoires avant d'appliquer la force de maintien à l'échantillon et, de préférence, dans lequel l'espacement préréglé est ajustable de manière sélective.

19. Ensemble de préhension selon l'une quelconque des revendications précédentes, dans lequel l'organe d'entraînement comporte une paire mutuellement opposée de faces de mise en prise (241), de préférence dans lequel chaque face de mise en prise (241) est inclinée par rapport à l'axe central dans une plage de 10° à 45°, et dans lequel chaque face de mise en prise (241) est configurée pour venir en prise par contact avec le porte-échantillon et ainsi pousser le porte-échantillon pour fournir la force de maintien.

**20.** Ensemble de préhension selon l'une quelconque des revendications précédentes, dans lequel l'organe noyau comporte un élément espaceur (727) disposé entre la deuxième portion d'extrémité et la portion de corps.

**21.** Ensemble de préhension selon l'une quelconque des revendications précédentes, dans lequel le logement est configuré pour se déplacer par rapport à l'organe noyau dans une première direction axiale sur une distance dans une plage de 1 mm à 10 mm et, de préférence, dans une plage de 2 mm à 7 mm.

**22.** Ensemble de préhension selon l'une quelconque des revendications précédentes, comprenant en outre un moyen de sollicitation configuré pour déplacer le logement par rapport à l'organe noyau dans une direction vers la deuxième portion d'extrémité.

**23.** Ensemble de préhension selon l'une quelconque des revendications précédentes, comprenant en outre un conduit (590) configuré pour raccorder fluidiquement un premier orifice (591), pouvant être connecté à une alimentation principale en air comprimé, à un deuxième orifice (592) qui débouche dans le vérin pneumatique.

**24.** Ensemble de préhension selon la revendication 23, dans lequel le premier orifice est fourni sur l'organe noyau au niveau de, ou proximal à, la première portion d'extrémité, et dans lequel le conduit s'étend le long de l'axe central de l'organe noyau jusqu'au deuxième orifice et, facultativement, dans lequel la tête de piston pneumatique est fournie sur un organe de tête, mobile par rapport à l'organe noyau, et dans lequel l'organe de tête comporte un tube agencé pour s'étendre à travers l'organe de tête depuis le deuxième orifice vers la première portion d'extrémité de manière à venir en prise de manière télescopique avec le conduit.

**25.** Ensemble de préhension selon la revendication 23, dans lequel le premier orifice est agencé sur l'organe noyau et aligné avec une ouverture s'étendant à travers le manchon monté autour de l'organe noyau, et dans lequel l'ouverture est configurée de sorte que le premier orifice reste accessible à travers l'ouverture à mesure que le logement se déplace par rapport à l'organe noyau.

**FIG. 1**

PRIOR ART

**FIG. 2**

**FIG. 3**

(a)

(b)

**FIG. 4**

**FIG. 5**

**FIG. 6**

300

321
325
366 339
337
373
366
365
370
334
336
335
333
320
332
322
340
371
337
363

**FIG. 7**

400

421
425
491
469
461
469a
491
469
469b
461
420
B
440
481

**FIG. 8**

**FIG. 9**

**FIG. 10**

FIG. 11

FIG. 12

**FIG. 13**

**FIG. 14**

**REFERENCES CITED IN THE DESCRIPTION**

**Patent documents cited in the description**

- CN 207689272 U
- JP H11160213 A

- US 2007107534 A1